# EUROPEAN PATENT SPECIFICATION

(11) **EP 1 876 271 B1**
(45) Date of publication and mention of the grant of the patent: **02.06.2010**
(21) Application number: 06745695.4
(22) Date of filing: 26.04.2006
(51) Int. Cl.: D01F 6/90, A61F 13/00, A61L 15/00, A61L 31/00, D02G 3/04, D02G 3/36

(54) **X-RAY CONTRAST FILAMENT, X-RAY CONTRAST COVERED FILAMENT, AND FIBER STRUCTURE USING SAID X-RAY CONTRAST FILAMENT AND/OR X-RAY CONTRAST COVERED FILAMENT**
RÖNTGENKONTRASTFILAMENT, RÖNTGENKONTRASTBESCHICHTETES FILAMENT SOWIE FASERGEBILDE MIT DEM RÖNTGENKONTRASTFILAMENT UND/ODER DEM RÖNTGENKONTRASTBESCHICHTETEN FILAMENT
FILAMENT ET FILAMENT RECOUVERT DE CONTRASTE DE RAYONS X ET STRUCTURE FIBREUSE UTILISANT LEDIT FILAMENT ET/OU FILAMENT RECOUVERT DE CONTRASTE DE RAYONS X

(30) Priority: 26.04.2005 JP 2005128510; 27.01.2006 JP 2006018314
(43) Date of publication of application: 09.01.2008
(73) Proprietor: Unitika Fibers LTD., Chuoh-ku Osaka-shi Osaka, 5410058 (JP)
(72) Inventor: ABE, Seiji c/o UNITIKA FIBERS LTD., Uji Plant, Kyoto 611-8555 (JP); KAKUMOTO, Koji c/o UNITIKA FIBERS LTD., Uji Plant, Kyoto 611-8555 (JP); DOMON, Takenori c/o UNITIKA FIBERS LTD., Uji Plant, Kyoto 611-8555 (JP); HORIMOTO, Kazutoyo c/o UNITIKA FIBERS LTD.,t, Kyoto 611-8555 (JP); CHIZUKA, Kenji c/o UNITIKA FIBERS LTD., Uji Plant, Kyoto 611-8555 (JP); SHIMONOMURA, Takeru c/o UNITIKA FIBERS LTD.,, Kyoto 611-8555 (JP)
(74) Representative: Pautex Schneider, Nicole Véronique
(86) International application number: PCT/JP2006/308717
(87) International publication number: WO 2006/115266

(56) References cited:
- EP-A- 0 101 650
- JP-A- 02 118 131
- JP-A- 2002 266 157
- JP-A- 2002 325 775
- JP-A- 2004 162 239
- JP-A- 2006 051 209
- US-A- 5 997 980

## Description

### Technical Field

The present invention relates to an X-ray opaque filament, an X-ray opaque covered filament and a fiber structure using the X-ray opaque filament and/or the X-ray opaque covered filament. The present invention particularly relates to an X-ray opaque filament and an X-ray opaque covered filament each of which is a fiber formed of a thermoplastic resin containing an X-ray opaque agent, able to be photographed by the use of X-ray, and suitably used in fabric such as woven fabric, knitted fabric or nonwoven fabric used in various medical purposes, and relates to a fiber structure such as woven fabric, knitted fabric and nonwoven fabric using the X-ray opaque filament and/or X-ray opaque covered filament.

### Background Art

It has recently been desired to develop a medical-purpose polymer material that can be photographed by the use of X-ray. For example, JP-A-2000-336521 proposes a hollow fiber or hollow monofilament containing an opaque medium in the hollow portion. This is because, in a conventional technique known in the art, it was impossible that a powdery opaque component such as barium sulfate is blended with a polymer material, melt-spun and drawn. Therefore, JP-A-2000-336521 proposes that a hollow fiber or hollow monofilament is formed, and thereafter, an opaque medium is injected into the hollow portion thereof. In addition, JP-A-2000-336521 describes that the hollow fiber or hollow monofilament is woven into a braid for use or cut into short fiber pieces for use in various types of medical members including a bone fixation material such as pins.

JP-A-2002-266157 describes the X-ray sensitive fiber formed of a thermoplastic resin containing an X-ray opaque agent, which cannot be obtained by melt-spinning and drawing in JP-A-2000-336521. In JP-A-2002-266157, the X-ray sensitive fiber is used by partly weaving it into cloth such as surgical gauze.

Such surgical gauze, if it is left in the body, can be found by introducing an X-ray opaque filament into part of a fiber constituting the fabric in advance. However, it is often difficult to find the surgical gauze left in the body by photograph using X-ray because of the presence of various organs and body fluid, etc. in the body. Therefore, the X-ray opaque filament has been desired to have a higher opaque property than ever.

However, in the fiber described in JP-A-2000-336521, since an opaque medium is injected only in the hollow portion of the fiber, the opaque property is insufficient. Also in the fiber described in JP-A-2002-266157, since the content of an X-ray opaque agent is not high, sufficient X-ray opaque performance cannot be obtained. Besides this, since no consideration is given to post-processability of these two fibers, when surgical gauze etc., is obtained by applying post-processing, for example, by weaving a fiber into the gauze, problems such as wrinkle and loss of the X-ray sensitive fiber alone are raised.

JP-A-2-118131 proposes a covered X-ray opaque filament, in which a core filament formed of polypropylene containing an X-ray opaque filler is covered with a sheath filament having a low degree of fineness than the core filament. In this fiber, since the core filament is covered with the sheath filament, the core filament looks in wavy form. Because of such specific form, when the fiber is observed under X-ray radiation, not a straight image but a different image is seen. The fiber can be clearly distinguished.

However, also in the covered X-ray opaque filament of JP-A-2-118131, the X-ray opaque performance thereof is insufficient. In addition, in JP-A-2-118131, no mention is made of application of the filament and no consideration is given to post-processability.

### Disclosure of the Invention

### Problem to be solved by the Invention

The present invention was attained by overcoming the aforementioned problems. An technical object of the present invention is to provide an X-ray opaque filament and X-ray opaque covered filament which is excellent not only in X-ray opaque performance but also in post-processability and capable of forming a product by weaving it into woven fabric and nonwoven fabric without occurrence of wrinkle and loss of a fiber from the product, and further provide a fiber structure which contains the X-ray opaque filament and/or the X-ray opaque covered filament.

### Means for Solving Problem

To attain the aforementioned object, the present invention provides an X-ray opaque filament formed of a thermoplastic resin containing an X-ray opaque agent, wherein the X-ray opaque filament has a dry heat shrinkage of 3.5 to 0% at 130°C.

In another The X-ray opaque filament of the present invention, which is a filament formed of a thermoplastic resin containing an X-ray opaque agent, wherein an oil containing an ionic surfactant component in a ratio of 0 to 10% by mass is added.

According to the present invention, in the X-ray opaque filament, it is preferable that the thermoplastic resin is nylon 12.

According to the present invention, it is preferable that the X-ray opaque filament consists only of a thermoplastic resin containing an X-ray opaque agent.

According to the present invention, it is preferable that the X-ray opaque filament is a monofilament having a degree of fineness within 1000 to 20000 dtex.

According to the present invention, it is preferable that the X-ray opaque filament is a multifilament having a total degree of fineness within 1000 to 20000 dtex and a degree of fineness per single filament within 20 to 400 dtex.

In an X-ray opaque covered filament of the present invention, an X-ray opaque filament formed of a thermoplastic resin containing an X-ray opaque agent is covered with covering filament and the X-ray opaque covered filament has a dry heat shrinkage of 3.5 to 0% at 130°C.

According to the present invention, in the X-ray opaque covered filament, it is preferable that the X-ray opaque filament mentioned above is used.

According to the present invention, in the X-ray opaque covered filament, it is preferable that the covering filament has a lower degree of fineness than the X-ray opaque filament.

In another X-ray opaque covered filament according to the present invention, the X-ray opaque filament is used and at least a part of the covering filament is formed of a second thermoplastic resin having a lower melting point than a first thermoplastic resin forming the X-ray opaque filament.

According to the present invention, in the X-ray opaque covered filament, it is preferable that the melting point of the second thermoplastic resin is 100°C or more and lower by 20°C or more than the melting point of the first thermoplastic resin.

According to the present invention, in the X-ray opaque covered filament, it is preferable that the covering filament is a conjugate filament formed of a core portion and a sheath portion, and the sheath portion of the conjugate filament is formed of the second thermoplastic resin.

The fiber structure of the present invention is formed of the X-ray opaque filament and/or the X-ray opaque covered filament.

### Effect of the Invention

The X-ray opaque filament and X-ray opaque covered filament of the present invention is formed of a filament containing a thermoplastic resin containing an X-ray opaque agent and has a dry heat shrinkage of 3.5 to 0% at 130°C. Therefore, when the X-ray opaque filament and X-ray opaque covered filament of the present invention are used in various types of materials such as woven fabric, knitted fabric, nonwoven fabric, and particularly, medical gauze, occurrence of wrinkle and deformation of a product, due to large shrinkage, can be prevented and, at the same time, a high quality product can be obtained.

Furthermore, the X-ray opaque filament of the present invention can be twisted. The X-ray opaque filament may be used as the X-ray opaque covered filament. Moreover, the X-ray opaque filament of the X-ray opaque covered filament can be twisted. In this way, it is possible that the X-ray opaque filament is less likely to fall out from a product. In addition, since the sectional shape of the filament is rendered to be round, excellent opaque performance is obtained. Therefore, the X-ray opaque filament and X-ray opaque covered filament can be suitably used in a medical material such as surgical gauze.

Furthermore, the X-ray opaque filament and X-ray opaque covered filament of the present invention are formed of a filament containing a thermoplastic resin containing an X-ray opaque agent and an oil in which an ionic surfactant component in a ratio of 0 to 10% by mass is added. Therefore, when the filaments are shaken in water, foams are less likely to generate even in the presence of oil. By virtue of this, when products such as woven fabric, knitted fabric and nonwoven fabric are obtained, a process for removing spinning oil, for example, washing, is not required. Furthermore, the filaments can satisfy a foaming test required for medical gauze. Therefore, the filaments are suitably applied to various types of medical usages.

In another type of X-ray covered filament according to the present invention mentioned above, an X-ray opaque filament formed of a filament constituting of a first thermoplastic resin containing an X-ray opaque agent is covered with covering filament and the covering filament is at least partly formed of a second thermoplastic resin having a lower melting point than the first thermoplastic resin. When a fiber structure is formed by partly using the X-ray opaque covered filament and subjecting the filament to heat processing, at least one portion of the covering filament covering the X-ray opaque filament can be melted and solidified to adhere to the filament constituting the fiber structure. Therefore, it is possible to prevent loss of the X-ray opaque filament from the fiber structure. In addition, since the sectional shape of the X-ray opaque filament is not deformed. Accordingly, the fiber structure excellent in opaque property can be obtained.

The filament structure of the present invention (products such as woven fabric, knitted fabric, nonwoven fabric, fiber ball and fiber laminate) comprises the X-ray opaque filament and/or the X-ray opaque covered filament of the present invention. Therefore, the fiber structure can be obtained with the excellent X-ray opaque property while preventing occurrence of wrinkle and deformation of the product. Furthermore, since the X-ray opaque filament is less likely to fall out from a product, the resultant product is excellent in quality and thus suitably applied to various medical uses.

### Brief Description of the Drawings

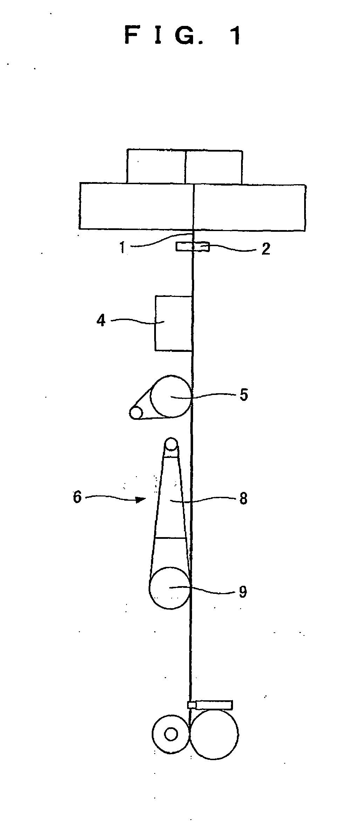

FIG. 1 is a schematic illustration of an apparatus for manufacturing an X-ray opaque filament according to the present invention.

### Best Mode for Carrying Out the Invention

The present invention will be described more specifically below.

An X-ray opaque filament and the X-ray opaque filament to be used in an X-ray opaque covered filament according to the present invention each are formed of a thermoplastic resin containing an X-ray opaque agent. As the thermoplastic resin, any thermoplastic resin may be used as long as a synthetic fiber can be obtained. Examples thereof include a polyamide, a polyester and a polyolefin. Of them, a polyamide is preferable. Examples of the polyamide include nylon 6, nylon 66, nylon 69, nylon 46, nylon 610, nylon 12 and polymethaxylene adipamide. The thermoplastic resin may be a copolymer or a mixture of these components. Of the polyamides, nylon 6 and nylon 12 are particularly preferable.

The reason why a polyamide is preferable as the thermoplastic resin is that a polyamide filament has excellent textures such as a soft texture and a moist texture derived from the feature of a polymer and such a texture is suitable for medical applications such as surgical gauze used in contact with an affected area. Furthermore, of the polyamides, nylon 12 is particularly preferable. This is because nylon 12, in addition to the aforementioned properties, can be melt-spun and drawn to make filaments even if an X-ray opaque agent is contained in a large concentration as described later.

When a polyester is used as the thermoplastic resin, for example, polyethylene terephthalate, polytrimethylene terephthalate, or polybutylene terephthalate may be used. When a polyolefin is used, for example, polypropylene or polyethylene may be used. These components may be used in the form of a copolymer, a mixture or the like.

A thermoplastic resin may be used singly or in a mixture of plurality of types.

Examples of the X-ray opaque agent to be contained in the thermoplastic resin include barium sulfate, bismuth subnitrate, tungsten oxide, thorium oxide and cesium oxide. Of them, barium sulfate is preferable. This is because it is excellent in X-ray impermeability and has high thermal resistance and crystal stability. In addition, since barium sulfate has a small primary particle size and it is possible to produce particles which are less likely to cause secondary aggregation, when barium sulfate is kneaded into a thermoplastic resin and melt-spun, filaments can be obtained with good workability accompanying no increase of filtration pressure and thread-cut dumpling, etc.

The particle size of the X-ray opaque agent is preferably large to some extent in view of improving opaque property. However, particles having an excessively large size are disadvantageous in view of dispersing them uniformly into filament. Conversely, particles having an excessively small size cause a problem of secondary aggregation. In consideration of the aforementioned points, the size of the primary particles of the X-ray opaque agent is preferably 0.5 to 10 µm, more preferably, 0.8 to 8 µm, and particularly preferably, 1.0 to 5 µm.

The X-ray opaque filament of the present invention is a filament formed of a thermoplastic resin containing an X-ray opaque agent. To improve opaque performance, it is preferable that the filament is formed of a single component, more specifically, formed only of a thermoplastic resin containing an X-ray opaque agent, in order to increase a resin portion having the X-ray opaque agent added thereto in the conditions where degree of fineness is equivalent. More specifically, a sheath/core conjugate filament containing an X-ray opaque agent only in the core portion is inferior to the single component filament in opaque property even if having the same degree of fineness as the single component fiber, because only the core portion has opaque property.

When a single component fiber is formed, it is preferable that an X-ray opaque agent is dispersed almost uniformly in a thermoplastic resin. To disperse an X-ray opaque agent in a thermoplastic resin almost uniformly, the X-ray opaque agent and the thermoplastic resin can be directly kneaded by use of an extruder or the like during a melt-spinning process. However, preferably, master chip, which contains the X-ray opaque agent in the large concentration, is prepared in advance, and then, the master chips are kneaded. This is because the master chips are kneaded more uniformly.

The X-ray opaque filament of the present invention can be used together with another type of filament to form various types of fiber structures such as woven fabric, knitted fabric, nonwoven fabric, fiber balls and fiber laminates. Of them, it is preferred to form fabric from the X-ray opaque filament of the present invention in combination with another type of filament to constitute, for example, woven fabric, knitted fabric and nonwoven fabric. The fabric is preferably used as surgical gauze. When woven or knitted fabric is formed, it is preferable that the X-ray opaque filament of the present invention is used together with another type of filament and partly integrated into the texture of the woven or knitted fabric in a weaving/knitting process. It is also preferable that after woven or knitted fabric consisting of another type of filament is produced, the X-ray opaque filament of the present invention is partly integrated in the texture. When nonwoven fabric is formed, it is preferable that a web formed of another type of filament is formed and thereafter, the X-ray opaque filaments of the present invention are arranged on the web and subjected, for example, to hydroentanglement processing to form nonwoven fabric.

When woven fabric or knitted fabric and nonwoven fabric, etc., are obtained by using the X-ray opaque filament of the present invention in combination with another type of filament as mentioned above, generally, a thermal setting process is required in order to improve strength and integration of the woven fabric, knitted fabric and nonwoven fabric or to dry them after the hydroentanglement processing. For example, when the filament of the present invention is used in spun-lace nonwoven fabric, thermal setting is preferably performed at a heat and dry state at 130°C. Therefore, the dry heat shrinkage under these conditions is a very important value in the present invention.

Accordingly, the X-ray opaque filament of the present invention preferably has a dry heat shrinkage (at 130°C) of 3.5 to 0%, more preferably, 2.0 to 0%, further preferably, 1.2 to 0% and still further preferably, 0.6 to 0%.

If the dry heat shrinkage is larger than 3.5%, the filament of the present invention greatly shrinks in a thermal setting process when it is used together with another type of fiber to form various type of materials such as woven fabric, knitted fabric and nonwoven fabric, with the result that a product gets wrinkled and deformed.

On the other hand, if the dry heat shrinkage is less than 0%, the filament is extended. Therefore, when the filament of the present invention is used together with another type of fiber to form, for example, woven fabric, knitted fabric and nonwoven fabric, the filament of the present invention gets loose in the product and sometimes falls out from the product.

In the present invention, the dry heat shrinkage at 130°C is measured as follows. That is, the X-ray opaque filament is rolled up to 10 rounds by use of a sizing reel of 1 m in length to form a hank, which is then controlled in moisture at 25°C, 65%RH for 24 hours. Next, a load (1/150 g) per dtex is applied to the ring of the hank, and the length (LO) at this time is measured. Furthermore, dry heat shrinkage treatment is performed under no application of load at 130°C for 30 minutes, and moisture is controlled at 25°C and 65%RH for 24 hours. Subsequently, load (1/150 g) per dtex is applied in the same manner as above, the length (L1) at this time is measured. The numerical values obtained above are fitted to the following equation to calculate a dry heat shrinkage. $Dry heat shrinkage \left(130⁢°C\right) \left(%\right) = \left[1-\left(L⁢1/L⁢0\right)\right] \times 100$

To reduce a dry heat shrinkage to 3.5% or less, hot drawing and relaxation heating treatment are preferably performed as shown particularly below in the case where the thermoplastic resin is nylon 6, nylon 12 or polypropylene; however, these treatments differ depending upon the type of thermoplastic resin. In this manner, the dry heat shrinkage of 3.5% or less can be attained.

The X-ray opaque filament of the present invention may be a monofilament or a multifilament. The X-ray opaque filament may be used as a long filament or as a short fiber by cutting it. In view of opaque property alone, a monofilament is preferable. However, when an X-ray opaque agent is added in a large concentration, a monofilament deteriorates in flexibility. In the usage requiring flexibility, a multifilament is preferable.

When the X-ray opaque filament of the present invention is used in fabric such as surgical gauze as described later, the X-ray opaque filament is required to have higher opaque performance. To improve the opaque performance, it is preferred to increase the content of the X-ray opaque agent in the filament.

The larger the content of the X-ray opaque agent in the X-ray opaque filament, the better in order to improve opaque performance. However, when the content is excessively large, fiber is broken at spinning or mechanical properties as a fiber may extremely deteriorate in some cases. In view of these, the content of the X-ray opaque agent in the filament is preferably 30 to 85% by mass, more preferably, 40 to 80% by mass, particularly preferably 60 to 78% by mass, and further preferably, 65 to 75% by mass.

When nylon 12 is used as a thermoplastic resin, even if a large amount of X-ray opaque agent is contained in the thermoplastic resin, melt spinning and drawing can be performed and a filament can be obtained with good workability.

The degree of fineness of a single X-ray opaque filament is a factor influencing opaque property. Therefore, in the case of a monofilament, the degree of fineness is preferably 1000 to 20000 dtex. In the case of a multifilament, the degree of fineness of a single filament is set at preferably 20 to 400 dtex and the degree of fineness of the multifilament is set at preferably 1000 to 20000 dtex.

To improve opaque property, either one of a monofilament and a multifilament (single filaments constituting the multifilament) is preferably a filament having a substantially circular sectional shape. Of the substantially circular shapes, a circle close to a complete round rather than an ellipse is preferable. When the sectional shape is an ellipse, the distances of some portions through which a beam of X-rays passes are shorter than those of other portions. In this case, opaque property may deteriorate. In contrast, when the sectional shape is a complete circle, the distances of the portions through which a beam of X-rays passes are equal. As a result, excellent opaque performance can be obtained.

In the case of a multifilament, the degree of fineness of a single filament is low compared to that of a monofilament. When the sectional shape of the multifilament is substantially circular, the same opaque performance as that of a monofilament can be obtained. To explain more specifically, when single filaments are unified to form a dense packing structure to form a multifilament, the sectional shape of the whole multifilament becomes virtually circular similarly to the sectional shape of a monofilament. As a result, the distance of a portion through which a beam of X-rays passes can be increased, providing good opaque performance. To keep a multifilament have a virtually circular sectional shape along with the lengthwise direction, it is preferred to twist the whole multifilament. The number of twists is preferably 20T/m or more, more preferably, 50T/m or more, and much more preferably, 60 to 120 T/m.

When the X-ray opaque filament is a multifilament throughout of which is twisted, integrity of the multifilament can be maintained, with the result that a single X-ray opaque filament is less likely to fall out from the product.

Examples of the X-ray opaque filament of the present invention may include an X-ray opaque filament having an oil added thereto. The X-ray opaque filament to which an oil is added has not any difference from known X-ray opaque filaments in the art. However, the X-ray opaque filament of the present invention greatly differs in the content of the added oil from X-ray opaque filaments known in the art. This is an important feature of the present invention. More specifically, in the oil added to the X-ray opaque filament of the present invention contains, the amount of an ionic surfactant component is low.

The ionic surfactant component refers to a cationic surfactant, an anionic surfactant and an amphoteric surfactant. Example of the cationic surfactant may include a quaternary ammonium salt. Examples of the anionic surfactant include an aliphatic acid salt, organic sulfonate salt, organic sulfate salt and organic phosphoric acid ester salt. Examples of the amphoteric surfactant include organic pedine and organic amine oxide.

The content of the ionic surfactant in the oil is preferably 0 to 10% by mass, more preferably, 0 to 6% by mass, and particularly preferably, 0 to 3% by mass. When an oil containing the ionic surfactant in excess of 10% by mass is added, the resultant X-ray opaque filament and a product produced from such a filament are likely to bubble when shaken in water.

More specifically, in the X-ray opaque filament of the present invention, the oil to be added contains an ionic surfactant within the range not exceeding 10% by mass, thereby satisfying the foaming test described in Appendix 4 of "Manual of Medical Nonwoven Gauze Standard", the Ministry of Health and Welfare, Notification No. 133 dated March 30, 2000 in Japan. When it is applied to various medical uses, a step of removing an oil such as a refining step is not required. On the other hand, the X-ray opaque filament to which an oil containing an ionic surfactant in excess of 10% by mass is added, fails to satisfy the aforementioned foaming test due to the surface activity of the ionic surfactant. Therefore, when such an X-ray opaque filament is applied to various medical uses, a step of removing an oil such as a refining step is required.

Note that the reason that a known oil contains a larger amount of ionic surfactant than that according the present invention is conceivably because an antistatic effect is improved by the presence of the ionic surfactant. In connection with this respect, the content of an ionic surfactant component is low in the present invention. Therefore, when it may be concerned that the antistatic effect of the oil is not sufficient, with the result that the property of unifying filaments during manufacturing deteriorates, and workability of filaments in transferring from step to step deteriorates, the antistatic effect of the oil can be improved by adding a nonionic surfactant. Examples of the nonionic surfactant include higher alcohols or alkyl phenols. Specific examples thereof include polyoxyethylene sorbitan fatty acid ester, fatty acid alkanolamide, polyoxyethylene alkyl ether, and polyoxyethylene alkylphenyl ether.

In the present invention, the amount of the oil added to the X-ray opaque filament is preferably 0.1 to 2.0% by mass based on the mass of the X-ray opaque filament, more preferably, 0.2 to 1.0% by mass, and particularly preferably, 0.3 to 0.7% by mass. When the amount of the oil is less than 0.1% by mass, for example, filaments cannot be sufficiently unified into a bundle, with the result that it tends to be difficult to spin filaments. On the other hand, when the content exceeds 2.0% by mass, for example, a roller may be contaminated with an oil during spinning, with the result that the operation is tends to be affected.

The X-ray opaque covered filament of the present invention is formed by covering an X-ray opaque filament formed of a thermoplastic resin containing an X-ray opaque agent with a covering filament and has a dry heat shrinkage of 3.5 to 0% at 130°C.

The X-ray opaque filament to be used in the X-ray opaque covered filament preferably has a dry heat shrinkage of 3.5 to 0% at 130°C and an oil containing an ionic surfactant component in a ratio of 0 to 10% by mass is preferably added to the filament. The thermoplastic resin used herein is preferably nylon 12. The X-ray opaque filament is preferably formed only of a thermoplastic resin containing an X-ray opaque agent. The X-ray opaque filament to be used in the X-ray opaque covered filament is preferably a monofilament having a degree of fineness of 1000 to 20000 dtex, or multifilament having a degree of fineness of 20 to 400 dtex per single filament and a degree of fineness of 1000 to 20000 dtex per whole filament.

The covering filament to be used in the X-ray opaque covered filament preferably has a low degree of fineness than the X-ray opaque filament. The material for the covering filament is not particularly limited and any one of a natural fiber, a synthetic fiber and others may be used. Examples of the natural fiber include cotton, hemp and silk thread. Examples of the synthetic fiber include filaments formed of a polyamide, polyester and polyolefin.

The X-ray opaque covered filament, by virtue of the presence of the covering filament covering the periphery of the X-ray opaque filament, can be easily entangled with another type of fiber constituting a product in the form of a fiber structure. Therefore, loss of the X-ray opaque filament from the product can be prevented. More specifically, loss of the X-ray opaque filament during not only manufacturing steps for obtaining a product but also use of the product can be prevented. Thus, the X-ray opaque covered filament can be used in various products and a high quality product can be obtained.

To prevent loss of an X-ray opaque filament from a product as mentioned above, in the case of a multifilament, the whole multifilament is preferably twisted, as mentioned above. By virtue of the presence of the twist in the surface of a multifilament, the multifilament can be easily entangled with another fiber or filament constituting a product.

However, in the X-ray opaque covered filament of the present invention, either a monofilament or a multifilament may be used as the X-ray opaque filament. However, in either case, the X-ray opaque filament is preferably covered with a covering filament so as to obtain a virtually circular sectional shape of the X-ray opaque covered filament. By virtue of this, even if a multifilament is used, an X-ray opaque covered filament having a virtually circular sectional shape can be obtained and provide the same opaque performance as that of a monofilament having a virtually circular section.

To obtain such an X-ray opaque covered filament, the covering filament that covers the X-ray opaque filament preferably has a lower degree of fineness than the X-ray opaque filament as is described above. Covering is preformed in the following manner. The X-ray opaque filament is preferably covered with the covering filament having a number of twists: 200 to 2000 T/m. The number of twists is preferably 500 T/m or more, and more preferably, 1000 T/m or more.

The number of twists of the covering filament and the degree of fineness of the covering filament per single filament and that of a unified filament of single filaments can be appropriately selected such that the sectional shape of a covered X-ray opaque filament is rendered to be substantially circular.

In the X-ray opaque covered filament of the present invention, it is preferable that the X-ray opaque filament itself is twisted. In this case, the number of twists of the X-ray opaque filament is preferably 2T/m or more, more preferably, 10T/m or more, and much more preferably, 20 to 50 T/m.

By virtue of using such an X-ray opaque filament twisted by itself is used, the X-ray opaque filament is less likely to fall out from the X-ray opaque covered filament, meaning that the X-ray opaque filament is less likely to fall out from a product. Furthermore, when the X-ray opaque filament is a multifilament, the integrity of a multifilament can be maintained. Thus, also in this case, a single X-ray opaque filament is less likely to fall out from the product.

In the X-ray opaque covered filament of the present invention, the dry heat shrinkage of the covering filament is not particularly limited. In contrast, the X-ray opaque covered filament is required to have a dry heat shrinkage of 3.5 to 0% at 130°C, preferably 2.0 to 0%, more preferably, 1.2 to 0%, and much more preferably, 0.6 to 0%.

The dry heat shrinkage of the X-ray opaque covered filament can be measured in the same manner as in the method of measuring a dry heat shrinkage of the X-ray opaque filament except that the X-ray opaque filament is replaced by the X-ray opaque covered filament.

In the X-ray opaque covered filament of the present invention, it is preferred to use an X-ray opaque filament to which the aforementioned oil is added. More preferably, the oil is added to not only the X-ray opaque filament but also the covering filament. Note that it is also preferable that the oil is added only to the covering filaments.

Next, another type of X-ray opaque covered filament of the present invention as mentioned above will be described in detail.

The X-ray opaque covered filament employs the X-ray opaque filament of the present invention. The covering filament thereof is at least partly formed of a second thermoplastic resin having a lower melting point than a first thermoplastic resin forming the X-ray opaque filament.

In this case, it is preferable that the melting point of the second thermoplastic resin constituting at least part of the covering filament is 100°C or more and lower by 20°C or more than the melting point of the first thermoplastic resin constituting the X-ray opaque filament. When the difference between the melting points is less than 20°C, the X-ray opaque filament itself may be melted depending upon the heat processing temperature during heat bonding process for obtaining a fiber structure. In contrast, when the melting point of the second thermoplastic resin is less than 100°C, the covering filament is possibly melted when the X-ray opaque covered filament and a fiber structure such as gauze containing the X-ray opaque covered filament are sterilized by heating.

When a fiber structure is formed by using such an X-ray opaque covered filament as mentioned above and subjected to heat processing, the second thermoplastic resin constituting the covering filament of the X-ray opaque covered filament is allowed to melt to adhere to another type of fiber or filament constituting the fiber structure. By virtue of this, it is possible to satisfactorily prevent loss of the X-ray opaque filament from the fiber structure. Since the second thermoplastic resin of the covering filament has a lower melting point than the first thermoplastic resin constituting the X-ray opaque filament, it is possible that only the second thermoplastic resin of the covering filament melts or softens but the thermoplastic resin constituting the X-ray opaque filament cannot melt during a heat processing. Therefore, it is possible to avoid deformation of the sectional shape of the X-ray opaque filament, with the result that a fiber structure excellent in opaque property can be obtained.

The covering filament is at least partly formed of a second thermoplastic resin; however, it may be a composite filament formed of the second thermoplastic resin and another type of thermoplastic resin or a single-component filament formed only of the second thermoplastic resin. However, it is preferable that at least the surface of the covering filament is formed of a second thermoplastic resin. Examples of the second thermoplastic resin include a polyolefin, a nylon-based copolymer and a polyester based copolymer. To allow the X-ray opaque filament to adhere tight to another type of fiber or filament constituting a fiber structure, the second thermoplastic resin preferably has good adhesion properties with both sides. For example, when the X-ray opaque filament is formed of nylon 12, a nylon-based copolymer may be preferably used as the low melting-point thermoplastic resin.

Examples of the polyolefin that can be used as the second thermoplastic resin may include polyethylene and polypropylene. In particular, a low-density polyethylene polymerized in the presence of a metallocene catalyst is preferable since it has a narrow molecular weight distribution and high resistance to e.g., thermal decomposition.

Examples of the nylon-based copolymer that can be used as the second thermoplastic resin may include a binary copolymer and ternary copolymer consisting of an arbitrary combination of elements including nylon 6, nylon 12, nylon 66 and nylon 610 or the like.

Examples of the polyester-based copolymer that can be used as the second thermoplastic resin may include a polyester-based copolymer obtained by copolymerization of a dibasic acid or at least one type of derivative thereof and at least one type of glycol. Examples of the dibasic acid that can be used herein include aromatic dibasic acids such as terephthalic acid, isophthalic acid, phthalic acid, p-oxybenzoic acid, 5-sodium sulfoisophthalic acid, and naphthalene dicarboxylic acid; aliphatic dibasic acids such as oxalic acid, adipic acid, sebacic acid, azelaic acid, and dodecane dicarboxylic acid; and alicyclic dibasic acids such as 1,2-cyclobutanecarboxylic acid. Examples of the glycol include ethylene glycol, diethylene glycol, triethylene glycol, propanediol, butanediol, pentanediol, hexanediol, neopentanediol, p-xylene glycol, and polyalkylene glycol such as polyethylene glycol, polytetramethylene glycol. Furthermore, a polyester copolymer obtained by copolymerization of an aromatic polyester and an aliphatic lactone may be preferably used. Examples of the aromatic polyester include a polymer of an ethylene terephthalate unit and/or a butylene terephthalate unit, or copolymers obtained by further copolymerizing isophthalic acid, 2,6-naphthalene dicarboxylic acid, adpic acid, sebacic acid, ethylene glycol, 1,6-hexanediol or the like to these. As the aliphatic lactone, lactones having 4 to 11 carbon atoms may be used singly or in combination of two or more types. Examples of a particularly preferable lactone include s-caprolactone and δ-valerolactone.

When a composite filament is used as the covering filament, a sheath/core conjugate filament is preferable in which the second thermoplastic resin as mentioned above is used in the sheath portion and another type of thermoplastic resin is used in the core portion. When the sheath/core conjugate filament is used as the covering filament, even if the sheath portion is melted to adhere to X-ray opaque filament and/or a filament constituting the fiber structure, the resin of the core portion is not melted and thereby the strength of the covering fiber can be maintained. Therefore, when the X-ray opaque filaments are bundled, loss of a single filament can be effectively prevented.

Examples of said another type of thermoplastic resin to be used when a conjugate filament is used as the covering filament include a polyamide, a polyester and a polyolefin. Examples of the polyamide include nylon 6, nylon 66, nylon 69, nylon 46, nylon 610, nylon 12 and polymethaxylene adipamide. Examples of the polyester include polyethylene terephthalate, polytrimethylene terephthalate, and polybutylene terephthalate. When a polyolefin is used, polypropylene, polyethylene or the like may be used. Furthermore, a copolymer or a mixture of these components may be used.

When a conjugate filament is used as the covering filament, the ratio (% by mass) of the second thermoplastic resin to the whole covering filament is preferably 10% or more, and more preferably, 20% or more. When the ratio of the second thermoplastic resin is excessively low, the ratio of the adhesion portion by a heat processing is low, with the result that X-ray opaque filament is likely to fall out from a fiber structure.

The heat processing for melting the second thermoplastic resin constituting the covering filament may be applied directly to the X-ray opaque covered filament alone or the X-ray opaque covered filament formed into a fiber structure such as fabric. In consideration of workability for forming a fiber structure such as fabric, the heat processing is preferably applied after the fiber structure is formed.

As a heat processor for melting the second thermoplastic resin constituting covering filament of an X-ray opaque covered filament, a general heat processing apparatus can be used. However, to keep the sectional shape of the X-ray opaque covered filament, a non-contact type dry heat processing apparatus such as a slit heater is preferably used. In this way, an X-ray opaque covered filament, in which at least one portion of the covering filament is melted to adhere to the X-ray opaque filament, can be obtained. When the second thermoplastic resin of the X-ray opaque covered filament is once melted, the resultant X-ray opaque covered filament is used to form a fiber structure such as woven fabric or nonwoven fabric, and then, heat processing is applied to the fiber structure, the second thermoplastic resin once melted and solidified is further melted again to adhere to the fiber structure. Therefore, loss of an X-ray opaque filament from the fiber structure can be prevented.

The fiber structure of the present invention will be described. The fiber structure of the present invention is constituted of the X-ray opaque filament and/or the X-ray opaque covered filament of the present invention, and more specifically, constituted by at least partly using the X-ray opaque filament and/or the X-ray opaque covered filament of the present invention. Specific examples of the fiber structure include fabric such as woven fabric, knitted fabric and nonwoven fabric, a fiber laminate and a fiber ball. Of them, fabric is preferable and woven and nonwoven fabric is more preferable. These woven fabric and nonwoven fabric contain the X-ray opaque filament and/or X-ray opaque covered filament of the present invention in combination of another type of fiber constituting the woven and nonwoven fabric. Therefore, the woven fabric and nonwoven fabric are excellent in opaque property and apparent quality. In addition, the X-ray opaque filament is less likely to fall out from the woven and nonwoven fabric.

When a surgical operation or the like is performed, many pieces of gauze are used in order to wipe and absorb, for example, blood and body fluid, of the patient. After the surgery, it is necessary to take out all pieces of gauze from the patient. However, gauze used in the surgery is stained red with blood, which is less likely to be distinguished from the organs of the patient at the incised portion. As a result, gauze is sometimes left in the body of the patient. When the gauze remains in the body for a long time, the patient feels physical pain and has fever. Moreover, the gauze adheres to an organ and likely causes other diseases. As a measure of preventing such incidents, gauze pieces are counted after the surgery. However, it is not easy work and takes time to count gauze pieces stained with blood. In addition, miscount may occur. Hence, this measure alone may not be sufficient.

Of the fiber structures of the present invention, fabric such as woven and nonwoven fabric mentioned above containing a filament having X-ray opaque property can be detected by using X-ray when the fabric is left in the body. In this way, all fabric pieces used in the surgery can be removed. Besides this, according to the present invention, the X-ray opaque filament having a low dry heat shrinkage is used. Therefore, even if heat is applied in a heat processing step during fabric manufacturing process, the resultant product has no wrinkle. The product can be obtained with good quality and suitably used as medical gauze. Furthermore, when an X-ray opaque covered filament formed by covering an X-ray opaque filament with another type of filament (covering filament) is used, loss of an X-ray opaque filament from the fabric can be prevented. Moreover, the sectional shape of the resultant filament becomes substantially circular. Hence, excellent opaque performance can be obtained.

Of the fiber structures of the present invention, first, woven fabric (plain woven fabric) will be described.

As the warp and the weft constituting woven fabric according to the present invention, any type of fiber such as a synthetic fiber, natural fiber or regenerated fiber may be used as long as it has fiber form, more specifically, as long as it has a structure such as a spun yarn formed of short fibers, a fiber bundle formed of one or more long filaments and a combination of these. Of these, a natural fiber such as cotton and a regeneration fiber such as solvent spun cellulose fiber, viscose rayon or cuprammonium rayon (Cupra rayon) has a relatively good water absorptivity, and therefore are suitable for wiping and absorbing blood and body fluid. The fibers constituting the woven fabric may be constituted of a single type of fiber and two types or more of fibers in combination as long as the object of the present invention is not lost.

The warp and weft constituting woven fabric are not particularly limited by a degree of fineness as long as it is used in plain weave fabric. For example, a pure cotton yarn such as cotton yarn count 40 may be used. When the woven fabric is used as medical gauze, the density of the yarn may fall within the range of those generally used as medical gauze. However, in view of the absorption amount and handling, both the ward and waft densities are preferably about 5 to 20 lines/cm.

The X-ray opaque filament and/or the X-ray opaque covered filament must be integrated in woven fabric having a flat texture. In weaving plain weave fabric, the X-ray opaque filament and/or the X-ray opaque covered filament may be woven as at least one of the warp and or at least one of the weft or may be inserted after the woven fabric is prepared.

The woven fabric containing the X-ray opaque filament and/or the X-ray opaque covered filament thus obtained may be laminated with another type of woven fabric and/or nonwoven fabric. The laminate can be subjected to hydroentanglement processing to integrate to each other and then put in use.

Next, nonwoven fabric of the fiber structures according to the present invention will be described.

The main fiber constituting the nonwoven fabric is preferably a non-thermoplastic fiber. This is because many thermoplastic fibers are poor in water absorptivity and thus are not suitable for wiping and absorbing blood and body fluid. Preferable examples of the non-thermoplastic fiber include a natural fiber such as cotton, which is relatively good in water absorptivity, and regeneration fibers such as a solvent spun sellulose fiber, viscose rayon or cuprammonium rayon (Cupra rayon). Of them, the solvent spun cellulose fiber is preferable. This is because it has high crystallinity, high orientation, high initial young modulus and high strength during wet time. The solvent spun cellulose fiber is obtained by spinning a raw-material solution in which cellulose is dissolved in a specific organic solvent without chemically modifying the cellulose or by spinning chips prepared by drying the raw-material solution. More specifically, the solvent spun cellulose fiber is sold by Lenzing under the name/trade name "Lenzing lyocell". The non-thermoplastic fiber constituting the nonwoven fabric may be constituted of a single type of fiber or constituted of two types or more of fibers in combination as long as the object of the present invention is not damaged.

A main fiber constituting nonwoven fabric preferably has a degree of fineness per single fiber of 0.8 to 3.5 dtex and more preferably, 1.0 to 3.0 dtex. If the degree of fineness is less than 0.8 dtex, transportability of the fiber deteriorates in a carding step of nonwoven fabric manufacturing process. In contrast, when the degree of fineness exceeds 3.5 dtex, entangling of mutual fibers becomes poor, with the result that the degree of entangling at the entangling point decreases. In addition, the length of fiber is preferably as short as 20 to 85 mm. When the length of fiber deviates from this range, the transportability of the fiber deteriorates in a carding step of a nonwoven fabric manufacturing process.

The weight per unit area of nonwoven fabric, which is a fiber structure of the present invention, is preferably 25 to 150 g/m². When the weight per unit area is less than 25 g/m², the absorption amount of blood or the like is not sufficient. Conversely, when the weight per unit area exceeds 150 g/m², the absorption amount increases; however, it becomes difficult to handle the nonwoven fabric at the time of surgical operation.

The X-ray opaque filament and/or X-ray opaque covered filament for the nonwoven fabric must be contained in an appropriate amount in the nonwoven fabric. For example, nonwoven fabric according to the present invention can be formed by forming webs of the main fiber constituting the nonwoven fabric, arranging the X-ray opaque filaments and/or X-ray opaque covered filaments between two web layers and subjecting the resultant construct to hydroentanglement processing. Alternatively, nonwoven fabric according to the present invention can be obtained by subjecting a single-layer web to hydroentanglement processing to obtain nonwoven fabric and arranging the X-ray opaque filament and/or X-ray opaque covered filament on the surface of the nonwoven fabric obtained and further subjecting the resultant construct to hydroentanglement processing.

When the nonwoven fabric is obtained as described above, generally, a thermal-setting process must be performed to improve the strength and integrity of the nonwoven fabric or for dehydration performed, for example, after hydroentanglement processing. For example, when the filament of the present invention is used in span-lace nonwoven fabric, the thermal-setting is performed in a dry and hot state of 130°C. Therefore, in the present invention, a dry heat shrinkage under such the conditions is very important value, as described above.

In a fiber structure according to the present invention having an X-ray opaque covered filament whose covering filament is formed of a second thermoplastic resin having a lower melting point than a first thermoplastic resin constituting the X-ray opaque filament, it is preferable that the second thermoplastic resin constituting at least part of the covering filament is melted to adhere to the fiber constituting the fiber structure.

As a device for melting the second thermoplastic resin constituting the covering filament of an X-ray opaque covered filament after the X-ray opaque covered filament is formed into the fiber structure and contained therein, a heat processing apparatus may be used. The X-ray opaque covered filament may be allowed to adhere to the fiber constituting the fiber structure by a method of melting a second thermoplastic resin by use of the heat processing apparatus. Examples of the heat processing method include a method of passing the fiber structure through a non-contact dry heat processing apparatus such as a slit heater and a heat press method using a heat roller such as emboss roller. However, in view of opaque property and flexibility, the non-contact dry heat processing apparatus is preferably used. In particular, when the melting point of the second thermoplastic resin constituting the covering filament is 130°C or less, the second thermoplastic resin is melted by thermal-setting performed in a dry state of 130°C during a nonwoven fabric manufacturing process. Therefore, the covering filament is allowed to adhere to the main fiber constituting the nonwoven fabric during the thermo-setting process.

A method of manufacturing the X-ray opaque filament (multifilament) of the present invention will be described.

As a method of integrating an X-ray opaque agent into a thermoplastic resin in the present invention, a predetermined amount of the X-ray opaque agent can be directly added to the thermoplastic resin in a melt-spinning process and kneaded by an apparatus such as an extruder. However, there is another method, in which master chips are previously prepared by adding the X-ray opaque agent to the thermoplastic resin in a high concentration, and then, the master chips and general thermoplastic resin chips are blended together and kneaded. This method is preferable since the X-ray opaque agent can be more uniformly dispersed.

To explain more specifically, the master chips containing the X-ray opaque agent and the thermoplastic resin are kneaded and melted by an extruder and melt-spun by extruding the molten resin through a spinning nozzle in accordance with a known method. The spinning temperature is preferably set within the range of (Tm + 10)°C to (Tm + 80)°C where Tm is the melting temperature of the thermoplastic resin containing the X-ray opaque agent. When the spinning temperature is excessively high, the thermoplastic resin causes thermal decomposition, rendering smooth spinning difficult; at the same time, the physical properties of the resultant filament tend to be poor. In contrast, when the spinning temperature is excessively low, residue such as an unmelted product is likely to remain.

The spun filament is cooled and solidified by applying cool air of 15 to 40°C. In this way, the filament is wound once up at a rate of 200 to 1500 m/minute without being substantially drawn.

The undrawn multifilament obtained by winding-up as mentioned above is subjected to heat drawing. In this case, the heat drawing is preferably performed by applying a drawing tension of 1.0 g/dtex or less while applying heating processing to the filament at a heat processing temperature of (Tm-150)°C to (Tm-50)°C for a heat processing time of 0.02 seconds or more.

When the heat processing time during the drawing is set at 0.02 seconds or more, sufficient calories can be provided. Furthermore, when the drawing tension is set at 1.0 g/dtex or less, uniform drawing can be made.

The heat processing time during the drawing is preferably set at 0.02 seconds or more as mentioned above, more preferably, 0.05 seconds or more, and further preferably, 0.07 seconds or more. The drawing tension is preferably set at 1.0 g/dtex or less as mentioned above, more preferably, 0.8 g/dtex or less, and further more preferably, 0.6 g/dtex or less.

The drawing speed is not particularly limited. However, to set the heat processing time at 0.02 seconds or more, the drawing speed is preferably set at 500 m/minute or less, and more preferably, 200 m/minute or less, and further preferably, 100 m/minute or less. In view of the productivity, the drawing speed is preferably set at 50 m/minute or more.

The drawing temperature will be described. Generally, drawing is performed between rollers. When drawing is performed between hot rollers, the roller temperature is preferably set at (Tm-150)°C to (Tm-50)°C. When drawing is performed by setting a heater between the rollers, the temperature of the heater is preferably set at (Tm-150)°C to (Tm-50)°C.

The heat processing time refers to the total time required for the multifilament to pass through a heating zone, which is set at within the temperature range, in a drawing step. More specifically, when preheating is performed, the time of passing through the preheating zone must be included.

The drawing rate is preferably 20 to 60% of a maximum drawing rate (which is the drawing ratio at which an undrawn multifilament is broken by drawing). When the drawing ratio deviates from this range, drawing is not enough or too much.

Immediately after or in a certain interval after the heat drawing, relaxation heat processing is preferably performed. The relaxation heat processing is preferably performed at a tensile stress of 0.5 g/dtex or less for 0.5 seconds or more within the temperature range of (Tm-100)°C to (Tm-30)°C.

When the relaxation heat processing is performed continuously after the heat drawing as mentioned above, the multifilament can be sufficiently drawn and contracted. As a result, the dry heat shrinkage (at 130°C) of the X-ray opaque filament of the present invention can be set at 3.5% or less.

The X-ray opaque filament (multifilament) of the present invention can be obtained by the manner as mentioned above or, if necessary, by twisting it by a known method.

A method of manufacturing the X-ray opaque covered filament of the present invention will be described.

The X-ray opaque covered filament can be obtained by covering the X-ray opaque filament obtained in the aforementioned manner with a covering filament. When the X-ray opaque filament is covered with a covering filament, covering is preferably performed such that the number of twists of the covering filament is to be 200 to 2000 T/m, more preferably, 500 to 2000 T/m, and particularly preferably, 1000 to 2000 T/m. When covering is performed, the number of twists of the covering filament and other conditions may be appropriately selected such that the cross-sectional shape of the X-ray opaque filament becomes substantially circular.

Another type of X-ray opaque covered filament according to the present invention, which is formed by covering a X-ray opaque filament with a covering filament that at least partly contains a second thermoplastic resin having a lower melting point than that of a first thermoplastic resin constituting the X-ray opaque filament, can be obtained by covering the X-ray opaque filament with the covering filament in the manner as mentioned above. The covering filament is obtained by melt-spinning the second thermoplastic resin in combination with another type of thermoplastic resin constituting the covering filament by use of a general composite spinning apparatus such that the covering filament is obtained, for example, in a sheath/core form, and drawing and heat processing the resultant filament in accordance with a conventional method.

A preferable method of manufacturing an X-ray opaque filament according to the present invention having an oil added thereto will be described. In this case, the X-ray opaque filament can be manufactured in the same manner as mentioned above. The filament obtained by melt-spinning is cooled and solidified by applying cool air and an oil may be added in accordance with a known method.

In the preferable method of manufacturing an X-ray opaque covered filament according to the present invention having an oil added thereto, for example, the X-ray opaque filament to which an oil is added as mentioned above may be used. When X-ray opaque covered filament using a covering filament having an oil also added thereto is obtained, the covering filament may be prepared previously in a separate step by adding an oil thereto by a known method.

A preferable method for manufacturing woven fabric (plain woven fabric), which is one of the fiber structures of the present invention, will be described.

The woven fabric of the present invention is manufactured using pure cotton yarn, for example, cotton yarn count 40, as the warp and the weft by means of, for example, a general gauze weaving machine. In the case where the X-ray opaque filament and/or the X-ray opaque covered filament is used in place of at least one of the ward or at least one of the weft, the X-ray opaque filament and/or the X-ray opaque covered filament may be integrated into woven fabric and fixed therein. In the case where the X-ray opaque covered filament, which is formed by covering the X-ray opaque filament with a covering filament at least partly containing a second thermoplastic resin having a lower melting temperature than a first thermoplastic resin constituting the X-ray opaque filament, is used, the X-ray opaque covered filament can be melted to adhere to cotton yarn by applying heat processing to the woven fabric obtained. Furthermore, when heat processing is performed by using a hot emboss roller or an ultrasonic welding apparatus to melt the X-ray opaque filament and/or X-ray opaque covered filament to adhere to cotton yarn, the filament can be fixed more tightly. Alternatively, the X-ray opaque filaments are arranged on the woven fabric formed of cotton yarn alone and subjected to heat processing by a hot emboss roller or an ultrasonic welding apparatus to melt the X-ray opaque filament to adhere to the cotton yarn. The obtained woven fabric is appropriately defatted, breached and sterilized to obtain gauze. The obtained gauze can satisfy the standard defined by the Japanese Pharmacopoeia.

The X-ray opaque filaments and/or the X-ray opaque covered filaments are preferably arranged on woven fabric at appropriate intervals in the machine direction (lengthwise direction) of a manufacturing process of woven fabric. More specifically, the filaments may be arranged at intervals of about 10 to 300 mm. The filaments may not only be arranged linearly but also be arranged in a wavy or zigzag fashion.

A preferable method of manufacturing nonwoven fabric, which is one of the fiber structures of the present invention, will be described.

First, a fiber web is prepared, which is formed by accumulating, for example, solvent spun cellulose fibers as a main fiber. As the fiber web, a card web may be used, which is obtained by supplying solvent spun cellulose fibers to a carding machine. When holes are desired in the fiber web, a mesh-form support formed of rough woven cloth having predetermined opening portions, may be used. Subsequently, X-ray opaque filaments and/or the X-ray opaque covered filaments are arranged at appropriate intervals on the fiber web. Further on the resultant structure, a fiber web formed by accumulating solvent spun cellulose fibers is laminated to obtain a laminate.

The fiber webs arranged on and under the X-ray opaque filaments and/or the X-ray opaque covered filaments may be the same or different, for example, in weight per unit area. The weight per unit area of the fiber web to be positioned on and under the filaments may be appropriately selected in consideration of the weight per unit area of the nonwoven fabric to be finally obtained; however, preferably about 10 to 100 g/m² each.

The X-ray opaque filaments and/or the X-ray opqaue covered filaments are preferably arranged on the fiber web at appropriate intervals in the machine direction (lengthwise direction) of a manufacturing process of a product. More specifically, the filaments may be arranged at intervals of about 10 to 300 mm. The filaments may not only be arranged linearly but also be arranged in a wavy or zigzag fashion.

To the laminate, which is obtained by laminating a first fiber web, X-ray opaque filaments and/or X-ray opaque covered filaments, and a second fiber web in this order, pressurized liquid flow such as pressurized water flow is applied. In this manner, an entanglement treatment of, for example, solvent spun cellulose fibers is performed. Fibers are mutually entangled by application of the pressurized liquid flow to obtain an entirely integrated fiber sheet. In addition, since solvent spun cellulose fibers are entangled with X-ray opaque filaments and/or the X-ray opaque covered filaments, X-ray opaque filaments and/or the X-ray opaque covered filaments can be fixed to the fiber sheet.

The pressurized water flow can be obtained by use of a spray apparatus in which spray nozzles having a pore size of 0.05 to 2.0 mm are arranged at intervals of 0.05 to 10 mm in a single line or in a plurality of lines in the direction (transverse direction) perpendicular to the machine direction of a product manufacturing line. More specifically, the pressurized water flow can be obtained by spraying water through the spray nozzles at a pressure of 1.5 to 40 MPa. When the aforementioned mesh-form support formed of rough woven cloth is used, constituent fibers move to opening portions of the mesh-form support while being entangled with each other. However since no fibers are present at the portion corresponding to the knuckle portions of the support, opening holes are formed. In this way, nonwoven fabric formed of a fiber sheet having holes can be obtained.

The openings of the mesh-form support can be determined depending upon the surface configuration of the nonwoven fabric to be obtained and presence or absence of holes. For example, when the mesh-form support is woven cloth having about 16 to 25 meshes, nonwoven fabric having not only a smooth surface but also opening holes can be obtained. When the mesh-form support is woven cloth having 25 meshes or more, opening holes are less likely to be formed. In particular, when woven cloth has meshes exceeding 40, the nonwoven fabric obtained has an extremely smooth surface and excellent in drape property. The size of meshes may be appropriately selected depending upon the requirements for the nonwoven fabric to be desired. Note that the term "mesh" refers to the number of lines per inch. For example, rough woven cloth having 25 meshes refers to one having 25 lines per inch.

The nonwoven fabric having the X-ray opaque filaments and/or the X-ray opaque covered filaments obtained by hydroentanglement processing is cut into pieces of an appropriate size to obtain the nonwoven fabric of the present invention, which can be used, for example, as medical gauze.

When the X-ray opaque covered filament, in which the periphery of an X-ray opaque filament is covered with a covering filament at least partly containing a second thermoplastic resin having a lower melting point than a first thermoplastic resin constituting the X-ray opaque filament, is used, the covering filament can be melted to adhere to a main fiber constituting nonwoven fabric in a thermal setting step carried out for dehydration after hydroentanglement processing.

### Examples

The present invention will be now more specifically described by way of examples below. Note that physical property values are measured and evaluated in the Examples and Comparative Examples as follows.

### (a) Dry heat shrinkage (dry heat shrinkage at 130°C)

The dry heat shrinkage of the obtained X-ray opaque filament was measured by the aforementioned method. Note that, in the following Examples and Comparative Examples, when the degree of fineness of the obtained X-ray opaque filament was 3800 dtex (28 filaments), the load (weight to be applied to the ring of a hank) was set at 507 g.

### (b) Evaluation of fiber structure (woven fabric/nonwoven fabric)

The obtained woven fabric and nonwoven fabric were evaluated for opaque property, wrinkle occurrence and loss of a filament, as follows.

### (Opaque property)

The obtained woven fabric and nonwoven fabric were photographed by an X-ray camera under shooting conditions: X-ray irradiation distance: 1 m, X-ray generation apparatus (anode: tungsten) having a tube voltage of 80 kV and a tube current of 400 mA, irradiation time: 0.063 seconds. The visibility of the X-ray opaque filament and/or the X-ray opaque covered filament was visually evaluated in accordance with the following 4 grades.

E: very clearly observed
G: clearly observed
M: slightly clearly observed
P: substantially not observed

### (Occurrence of wrinkle)

The state of wrinkle appearing in the woven fabric and nonwoven fabric was visually evaluated in accordance with the following 5 grades.

1. The fabric is not wrinkled and the quality is good
2. The fabric is partly wrinkled but the quality is good
3. The fabric is entirely and slightly wrinkled but the quality is good
4. The fabric is entirely and somewhat wrinkled and no practical problem is observed in quality
5. The fabric is severely wrinkled and the quality is low.

### (Loss of a filament)

The obtained woven fabric and nonwoven fabric were cut into pieces. The X-ray opqaue filament and/or the X-ray opaque covered filament (both multifilament and single filament) were pulled and removed by hand from the cut edge thereof. The degree of easiness in removing a filament was evaluated in accordance with the following 4 grades.

1. When pulled strongly, neither a multifilament nor a single filament thereof is removed.

2. Neither a multifilament nor a single filament thereof is removed

3. Although a multifilament is not removed but a single filament thereof is removed more or less.

4. Both a multifilament and a single filament thereof are removed more or less.

### (c) Relative viscosity

Nylon 6: Viscosity was measured in accordance with a conventional method using 96% sulfuric acid as a solvent at a concentration of 1g/dl and a temperature of 25°C.

Nylon 12: Viscosity was measured in accordance with a conventional method using metacresol as a solvent at a concentration of 0.5 g/dl and a temperature of 25°C.

Polyethylene terephthalate: Viscosity was measured using a solvent mixture containing phenol and tetrachloroethane in equivalent amounts as a solvent at a sample concentration of 0.5 g/100 cc and a temperature of 20°C, by means of Ubbelohde viscometer.

### (d) Foaming test

An X-ray opaque filament or X-ray opaque covered filament (10 g) was washed while stirring in 1.5 L of water of 25°C for 5 minutes three times (1.5 L x 3) and dried at room temperature. The resultant filament was placed in a hard glass container having an inner volume of about 300 mL. To the container, 200 mL of water was accurately added. After closed tight with a tap, the container was heated in a pressurized vapor sterilizer at 121°C for one hour. Thereafter, the hard glass container was taken out from the pressurized vapor sterilizer and allowed to stand still until it reached room temperature. The resultant solution was used as a sample solution. About 5 mL of the sample solution was taken, placed in a test tube with a tap of 15 mm in inner diameter and about 200 mm in length, vigorously shaken for 3 minutes and allowed to stand still. The surface state of the solution was visually observed. The sample whose foams disappeared in 10 minutes was evaluated as G (acceptance), whereas the sample whose foams did not disappear in 10 minutes was evaluated as P (rejection).

### (e) Amount of Oil Pick Up (OPU)

(i) The mass (A0) of a conical flask dried at 105°C was measured.

(ii) 10 g of a test sample (the X-ray opaque filament or the X-ray opaque covered filament obtained) was taken, placed in the conical flask, dried by a hot air circulation dryer of 65°C for 1.5 hours, and cooled in a desiccator until it reached room temperature. After cool, the mass (A1) of the conical flask was measured. The mass of the sample was calculated in accordance with the equation: A1-A0.

(iii) To the conical flask (ii) housing the sample, n-hexane (60 to 70 mL) was added until the sample was sufficiently soaked. The flask was tapped tight and shaken at 40°C for 6 minutes to extract an oil.

(iv) The sample was taken out from the conical flask and washed with 15 to 20 mL of n-hexane. Thereafter, the sample was squeezed to remove n-hexane. N-hexane was collected including n-hexane used in washing and placed in the conical flask used above.

(v) The conical flask containing n-hexane was soaked in a water bath of 96 to 100°C to vaporize/evaporate n-hexane within the conical flask completely. Thereafter, the conical flask was dried for 2 hours in a hot air circulation dryer of 105°C and allowed to cool to room temperature in a desiccator. After cool, the mass (A2) of the conical flask was measured and OPU was calculated in accordance with the following equation.

$OPU \left(%\right) = \left(A⁢2-A⁢0\right) / \left(A⁢1-A⁢0\right) \times 100$

### (f) Weight of unit area of nonwoven fabric was measured in accordance with the description of JIS L 1906.

### [Examples/Comparative Examples of X-ray opaque filament and the X-ray opaque covered filament]

### (Example 1)

Chips of nylon 12 (VESTAMIDL 1900, manufactured by Daicel Degussa Ltd.) having a relative viscosity of 1.90 were prepared so as to contain 60% by mass of barium sulfate in a filament, supplied to a melt extruder, melted at a spinning temperature of 250°C, extruded from a spinning nozzle having 28 spinning holes of 0.50 mm in diameter. The undrawn filament was rolled up at a winding speed of 400 m/minute.

Subsequently, the obtained undrawn filament was subjected to hot drawing and relaxation heat processing under hot drawing/relaxation heat processing conditions shown in Table 1 in accordance with the process chart shown in FIG. 1. To explain more specifically, as shown in FIG. 1, an undrawn filament 1 was first pulled by a pulling roller 5 downward through a guide roller 2 and treated with heat by a box heater 4 provided below the guide roller 2. At this time, the temperature (heat processing temperature) of the box heater 4 was set at 150°C and the heat processing time was set at 0.09 seconds. Drawing (a draw ratio of 1.2 fold) was performed between the guide roller 2 and the pulling roller 5 while applying a tension (drawing tension) of 0.42 g/dtex to the undrawn filament. Subsequently, the relaxation heat processing was performed in a heat processing apparatus 6 having a saddle type plate heater 8 and a heat roller 9. The relaxation heat processing was performed while applying a tension of 0.04 g/dtex at a heat processing temperature of 150°C for a heat processing time of 3.8 seconds. The filament passed through the heat processing apparatus 6 was wound up to obtain an X-ray opaque filament of 3800 dtex/28f.

### (Examples 2 to 5 and 25 to 28, Comparative Examples 1 and 2)

Spinning, drawing, and relaxation heat processing were performed in the same manner as in Example 1 except that the content of barium sulfate in a filament was changed to each of the contents shown in Table 1 and the hot drawing/relaxation heat processing conditions were changed to obtain the values shown in Table 1, to obtain an X-ray opaque filament of 3800 dtex/28f.

### (Examples 6-11 and Comparative Examples 3 and 4)

The X-ray opaque filaments obtained in Examples 1 to 5 and Comparative Examples 1 and 2 and a polyester multifilament formed of polyethylene terephthalate of 84 dtex/36f serving as a covering filament were used. The covering filament was turned around the X-ray opaque filament by use of a covering twister so as to obtain the number of S-shaped twist shown in Table 1 to obtain an X-ray opaque covered filament. Other manufacturing conditions were as shown in Table 1.

### (Examples 12 and 13 and Comparative Example 5)

Spinning, drawing, and relaxation heat processing were performed in the same manner as in Example 1 except that the content of barium sulfate in a filament was changed to each of the contents shown in Table 1 and the hot drawing/relaxation heat processing conditions were changed to obtain the values shown in Table 1 to obtain filaments. The filament obtained was wound up. Subsequently, the filament was twisted by a ring twister as shown in Table 1 to form an X-ray opaque filament of 3800 dtex/28f.

### (Examples 14 and 15 and Comparative Example 6 and 7)

Spinning, drawing, and relaxation heat processing were performed in the same manner as in Example 1 except that the content of barium sulfate in a filament was changed to each of the contents shown in Table 1 and the hot drawing/relaxation heat processing conditions were changed to obtain the values shown in Table 1 to obtain a filament. The filament obtained was wound up. Subsequently, the filament was twisted by a ring twister as shown in Table 1 to form an X-ray opaque filament of 3800 dtex/28f.

Subsequently, an X-ray opaque covered filament was obtained by use of a covering twister in the same manner as in Example 6.

### (Examples 16 and 17)

Spinning, drawing, and relaxation heat processing were performed in the same manner as in Example 3 except that the X-ray opaque agent was changed to bismuth subnitrate (Example 16) and tungsten oxide (Example 17) and the content of the X-ray opaque agent in a filament to each of the contents shown in Table 1, to obtain an X-ray opaque filament of 3800 dtex/28f.

Subsequently, an X-ray opaque covered filament was obtained by a covering twister in the same manner as in Example 6.

### (Example 18)

Master chips were prepared using nylon 6 having a relative viscosity of 2.40 such that the content of barium sulfate in a filament was 55% by mass, supplied to extruder-type melt spinning machine, melted at a spinning temperature of 255°C, extruded from a spinning nozzle having 28 spinning holes of 0.50 mm in diameter. The undrawn filament was wound up at a winding speed of 400 m/minute.

Subsequently, the obtained undrawn filament was subjected to hot drawing/relaxation heat processing machine which was the same as used in Example 1 and hot drawing and heat processing were performed under the hot drawing/relaxation heat processing conditions as shown in Table 1 to obtain an X-ray opaque filament of 3800 dtex/28f.

### (Example 19 and Comparative Example 8)

Spinning, drawing, and relaxation heat processing were performed in the same manner as in Example 18 except that the content of barium sulfate in a filament was changed to each of the contents shown in Table 1 and the hot drawing/relaxation heat processing conditions were changed to obtain the values shown in Table 1, to obtain an X-ray opaque filament of 3800 dtex/28f.

### (Examples 20 and 21 and Comparative Example 9)

Spinning, drawing, and relaxation heat processing were performed in the same manner as in Example 18 except that the content of barium sulfate in a filament was changed to each of the contents shown in Table 1 and the hot drawing/relaxation heat processing conditions were changed to obtain the values shown in Table 1, to obtain an X-ray opaque filament of 3800 dtex/28f.

Subsequently, an X-ray opaque covered filament was obtained by a covering twister in the same manner as in Example 6.

### (Example 22)

Spinning, drawing, and relaxation heat processing were performed in the same manner as in Example 18 except that the content of barium sulfate in a filament was changed to each of the contents shown in Table 1 and the hot drawing/relaxation heat processing conditions were changed to obtain the values shown in Table 1 to obtain a filament. The filament obtained was rolled up. Subsequently, the filament was twisted by a ring twister as shown in Table 1 to form an X-ray opaque filament of 3800 dtex/28f.

### (Example 23)

Spinning, drawing, and relaxation heat processing were performed in the same manner as in Example 18 except that the content of barium sulfate in a filament was changed to each of the contents shown in Table 1 and the hot drawing/relaxation heat processing conditions were changed to obtain the values shown in Table 1 to obtain a filament. The filament obtained was wound up. Subsequently, the filament was twisted by a ring twister as shown in Table 1 to form an X-ray opaque filament of 3800 dtex/28f.

Subsequently, an X-ray opaque covered filament was obtained by a covering twister in the same manner as in Example 6.

### (Example 24)

Master chips were prepared using polypropylene chips (J107G, manufactured by Mitsui Chemicals Inc.) having a melt flow rate defined in JIS K7210 of 7 g/10 minutes such that the content of barium sulfate in a filament was 60% by mass, supplied to extruder-type melt spinning machine, melted at a spinning temperature of 230°C, extruded from a spinning nozzle having 28 spinning holes of 0.50 mm in diameter. The undrawn filament was wound up at a winding speed of 400 m/minute.

Subsequently, the obtained undrawn filament was subjected to hot drawing/relaxation heat processing machine which is the same as used in Example 1 and hot drawing and heat processing were performed under the hot drawing/relaxation heat processing conditions shown in Table 1 to obtain an X-ray opaque filament of 3800 dtex/28f.

Subsequently, an X-ray opaque covered filament was obtained by a covering twister in the same manner as in Example 6.

### (Comparative Example 10)

An X-ray opaque covered filament was obtained by a covering twister in the same manner as in Example 6 except that spinning was performed in the same manner as in Example 24, undrawn filament wound up was not drawn, and the number of twists of the covering fiber was changed to that shown in Table 1.

Physical property values of the X-ray opaque filaments and X-ray opaque covered filaments according to Examples 1 o 28 and Comparative Examples 1 to 10 obtained as mentioned above are shown in Table 1.

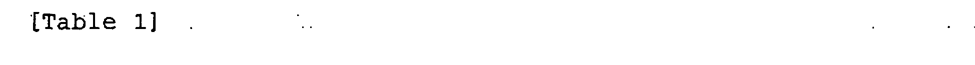

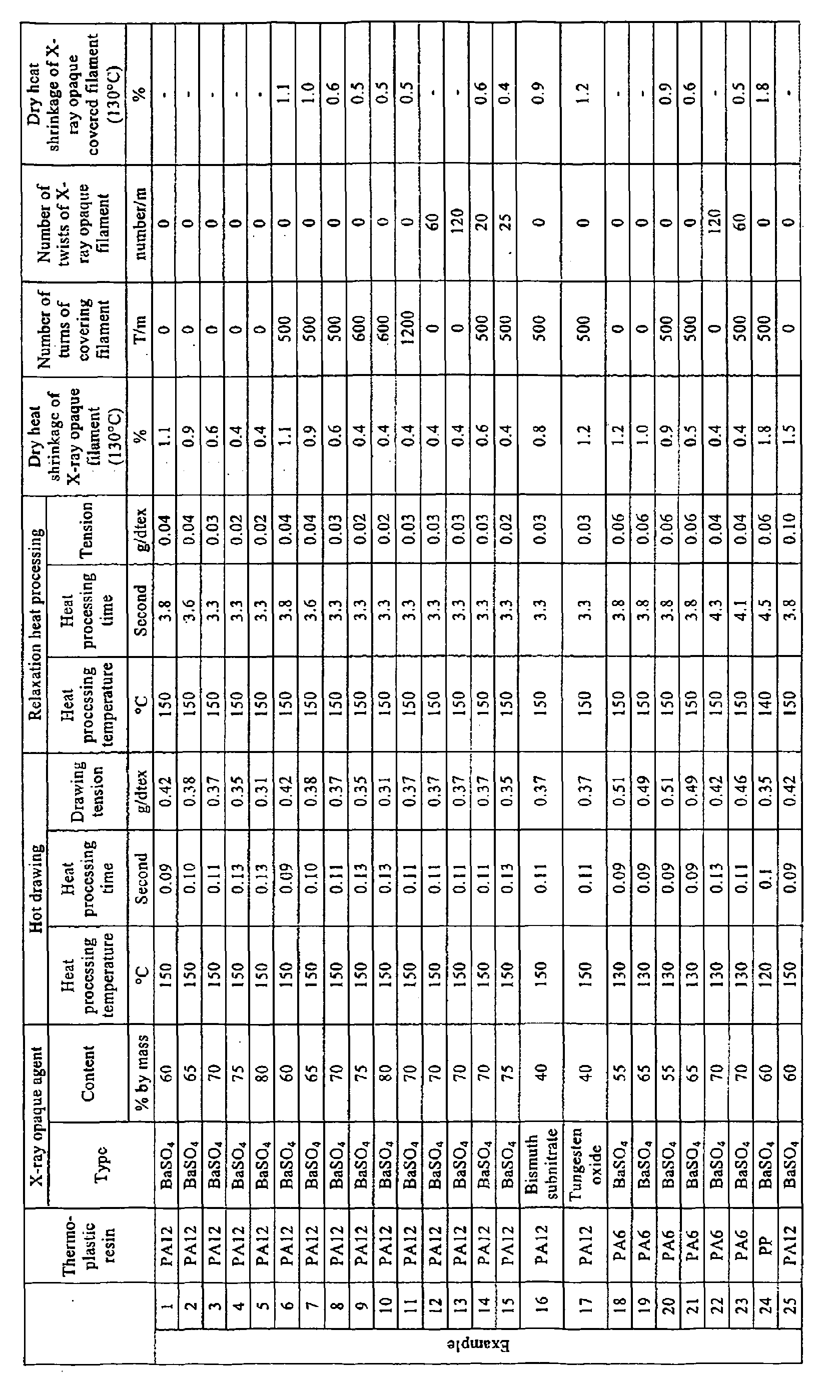

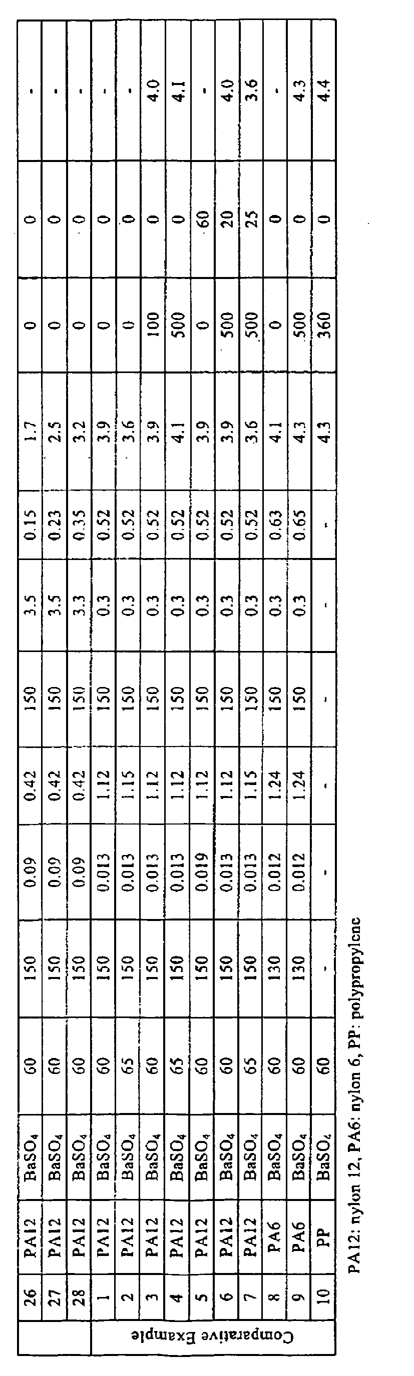

### [Examples and Comparative Examples of X-ray opaque filament and X-ray opaque covered filament having an oil added thereto]

### (Example 29)

Chips of nylon 12 (ESTAMIDL 1900, manufactured by Daicel Degussa Ltd.) having a relative viscosity of 1.90 and chips of the same type of nylon 12 containing barium sulfate in a high concentration were used and supplied to a melt extruder such that the content of barium sulfate in the whole chips was 60% by mass, melted at a temperature of 250°C, extruded from a spinning nozzle having 28 spinning holes of 0.50 mm in diameter. To the resultant filament, an oil having a composition (% by mass) shown in Table 2 was added. The filament was wound up at a winding speed of 400 m/minute to obtain an undrawn filament.

Subsequently, the obtained undrawn filament was subjected to the hot drawing and relaxation heat processing in accordance with the steps shown in FIG. 1 in the same manner as in Example 1 under the hot drawing/relaxation heat processing conditions shown in Table 2. The filament to which hot drawing and relaxation heat processing were applied was rolled up from the out port of the heat processing apparatus 6 to obtain X-ray opaque filament (not twisted), which is a multifilament of 3800 dtex/28f.

Subsequently, the solvent spun cellulose fibers (degree of fineness per single filament: 1.7 dtex, fiber length: 38 mm, brand name/trade name: "Lenzing lyocell" manufactured by Lenzing) was opened in a random carding machine to obtain a fiber web of about 15 g/m². The X-ray opaque filaments obtained above were arranged linearly on the fiber web in the machine direction (lengthwise direction) at intervals of 100 mm. Further on the filaments, the same web of about 15 g/m² obtained in the above was laminated to obtain a laminate.

The obtained laminate was placed on a mesh-form support having 100 meshes and treated twice by a spray apparatus in which spray nozzles having a pore size of 0.1 mm were arranged transversely in a single line at intervals of 0.6 mm at a spray pressure of 6.9 MPa. Subsequently, the laminate was turned upside down and the rear surface was treated by the spray apparatus twice at a spray pressure of 9.8 MPa. The laminate was further turned upside down and placed on a mesh-form support having 25 meshes and treated by the spray apparatus twice at a spray pressure of 9.8 MPa to obtain nonwoven fabric having a weight per unit area of 33 g/m².

### (Examples 30 and 31 and Comparative Examples 11 to 14)

An X-ray opaque filament was obtained in the same manner as in Example 29 except that the composition of an oil, the content of barium sulfate and hot drawing/relaxation heat processing conditions were changed to obtain the values shown in Table 2. Note that, in Comparative Examples 13 and 14, a covering filament was turned around the obtained X-ray opaque filament so as to obtain the number of S-shaped twists shown in Table 2 by use of a covering twister to obtain an X-ray opaque covered filament.

Subsequently, nonwoven fabric was obtained in the same manner as in Example 29 by using the X-ray opaque filament obtained.

### (Example 32)

An X-ray opaque covered filament was obtained using the X-ray opaque filament obtained in Example 31 and using polyester multifilament of 84 dtex/36f formed of polyethylene terephthalate and having the oil having the composition shown in the column "Example 32" of Table 2 added thereto, as the covering filament, more specifically, by turning the covering filament around the X-ray opaque filament so as to obtain the number of S-shaped twists of 500T/m.

Subsequently, nonwoven fabric was obtained in the same manner as in Example 29 using the X-ray opaque covered filament obtained.

The evaluation results of the X-ray opaque filaments, X-ray opaque covered filaments, and nonwoven fabric obtained in Examples 29 to 32 and Comparative Examples 11 to 14 are shown in Table 2.

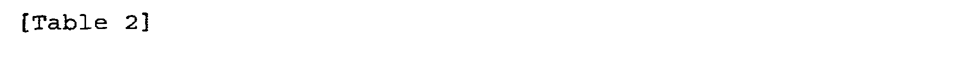

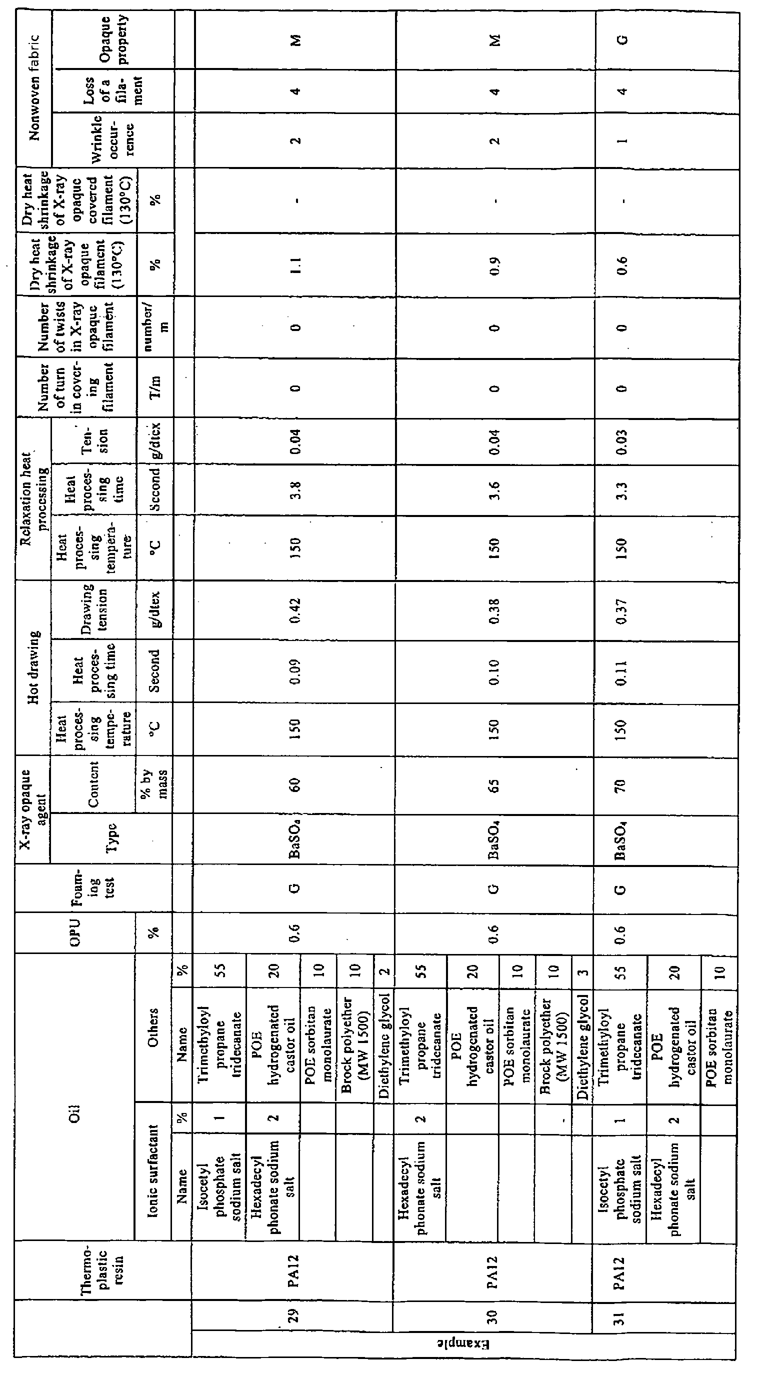

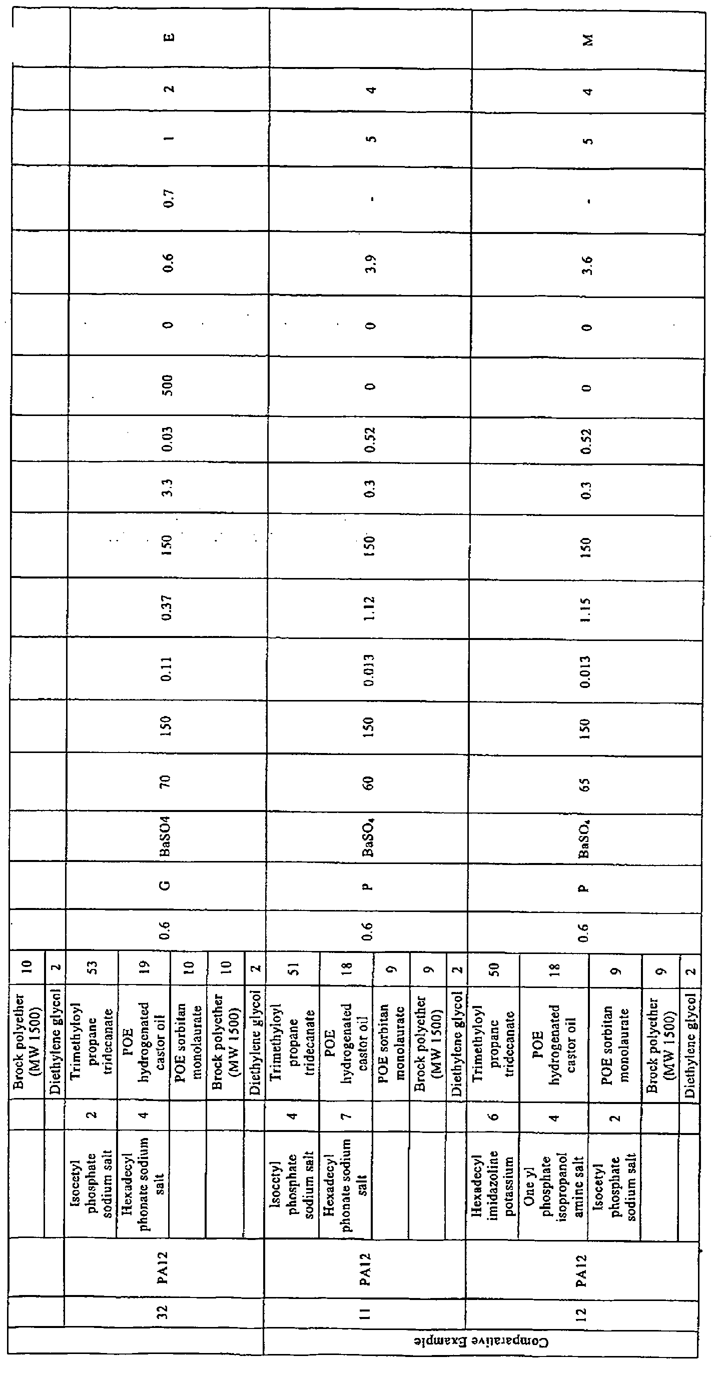

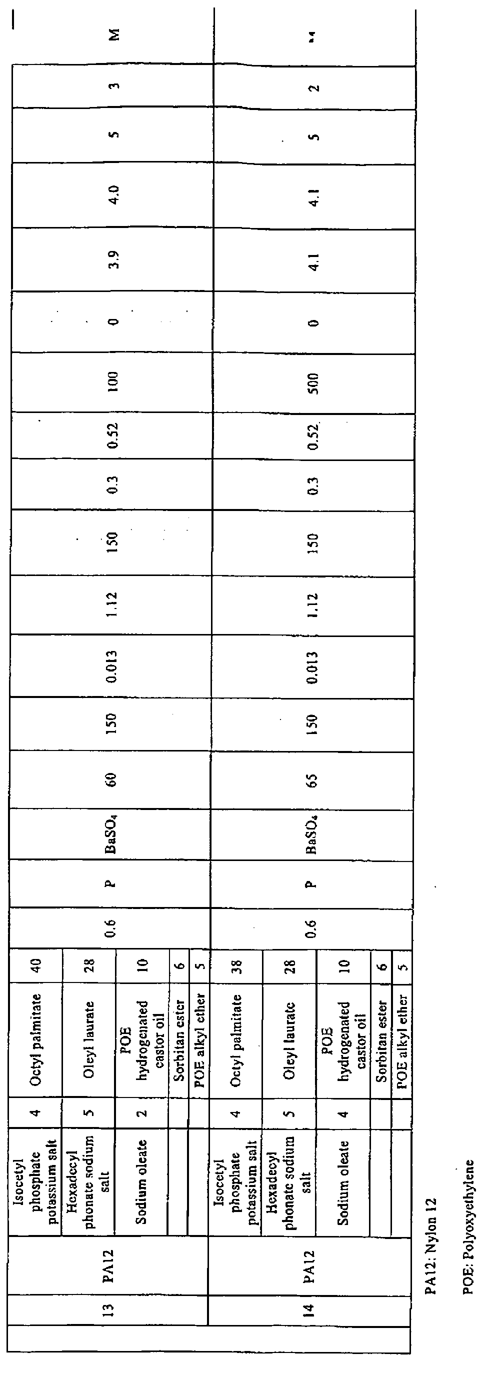

As is apparent from Table 2, in Examples 29 to 32, since the content of an ionic surfactant in the oil added thereof was 10% or less, foams disappeared within 10 minutes in a foaming test for the X-ray opaque filament and X-ray opaque covered filament. These filaments satisfied the object of the present invention. The nonwoven fabric obtained had neither wrinkle nor loss of a filament and good opaque property.
On the other hand, in the X-ray opaque filaments of Comparative Examples 11 to 14, since the content of an ionic surfactant in the oil added thereto exceeded 10%, foams did not disappear within 10 minutes in the foaming test. The nonwoven fabric obtained has many wrinkles and the quality in view of a product was low.

### [Examples and Comparative Examples of woven fabric]

### (Example 33)

Woven fabric (plain woven fabric) of 30 cm in width was obtained by using cotton yarn of yarn count 40 as the warp and weft such that 12 warps and wefts were contained per cm². On the woven fabric, the single X-ray opaque filament obtained in Example 5 was placed in parallel to the warp. The resultant construct was subjected to heat processing applied by an embossing apparatus to weld the X-ray opaque filament to the woven fabric to fix it.

Note that the embossing apparatus has a bumpy roll having scattered projections, which occupied a ratio of 15% to the whole area of the roll and were heated to a temperature of 235°C.

### (Example 34)

Woven fabric (plain woven fabric) was obtained in the same manner as in Example 33 except that one of the warps was replaced with the X-ray opaque filament obtained in Example 4 in place of placing a single X-ray opaque filament on the fabric and bonding it by heat processing to fix it.

### (Example 35)

Woven fabric (plain woven fabric) was obtained in the same manner as in Example 33 except that one of the warps was replaced with the X-ray opaque filament obtained in Example 3 in place of placing a single X-ray opaque filament on the fabric and bonding it by heat processing to fix it. The woven fabric was subjected to heat processing applied by an embossing apparatus to bond the X-ray opaque filament to the woven fabric to fix it in the same manner as in Example 33.

### (Examples 36 to 60 and Comparative Examples 15 to 24)

Woven fabric (plain woven fabric) was obtained in the same manner as in Example 34 except that one of the warps was replaced with the X-ray opaque filament or the X-ray opaque covered filament (obtained in each of Examples) shown in Table 3.

The physical property values and evaluations of woven fabric samples of Examples 33 to 60 and Comparative Examples 15 to 24 obtained as described above are shown in Table 3.

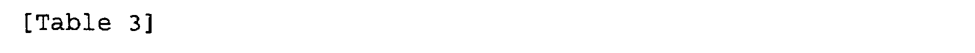

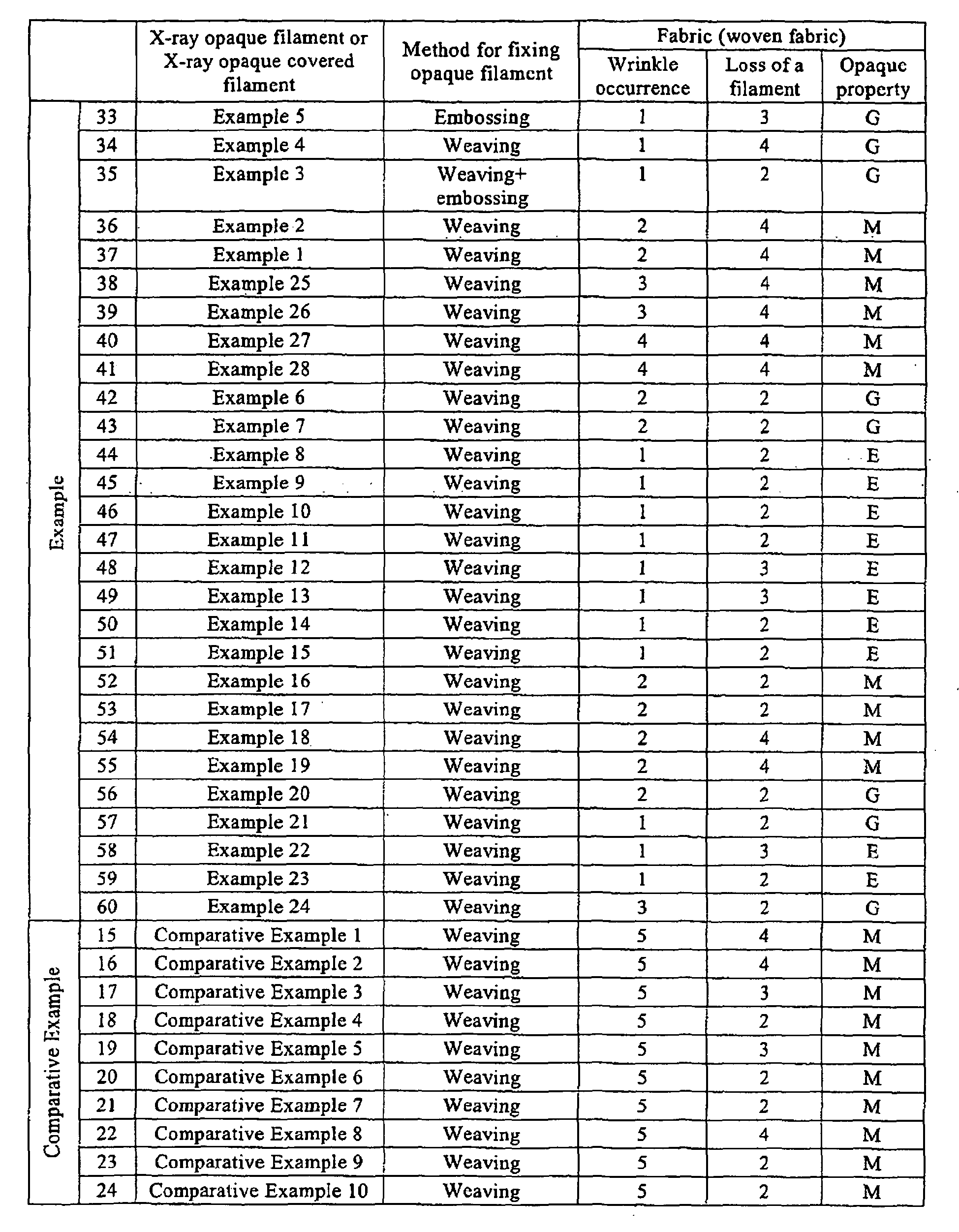

As is apparent from Table 3, in the X-ray opaque filaments or X-ray opaque covered filaments according to Examples 33 to 60, since the dry heat shrinkage of each of the filaments was 3.5% or less, the woven fabric samples obtained by using the filaments had neither wrinkle occurrence nor loss of a filament and satisfactory opaque property. In particular, the X-ray opaque covered filaments of Examples 42 to 47, 50 to 53, 56, 57, 59 and 60 had less loss of the X-ray opaque filaments since the X-ray opaque filaments were covered. In addition, since the X-ray opaque covered filaments were integrally formed such that the sectional shape of a multifilament is substantially circular, the woven fabric samples obtained by using these filaments had more excellent X-ray opaque property.

On the other hand, in each of the X-ray opaque filaments or X-ray opaque covered filaments according to Comparative Examples 15 to 24, since the dry heat shrinkage (at 130°C) of the X-ray opaque filament exceeded 3.5%, the woven fabric samples obtained by using these had many wrinkles and the quality in view of a product was low.

### [Examples and Comparative Examples of nonwoven fabric]

### (Example 61)

Solvent spun cellulose fiber A (degree of fineness per single filament: 1.7 dtex, fiber length: 38 mm, brand name/trade name: "Lenzing lyocell" manufactured by Lenzing) was opened in a random card to obtain a fiber web of about 15 g/m². The X-ray opaque filaments obtained in Example 5 were arranged linearly on the fiber web at intervals of 100 mm in the machine direction (lengthwise direction). Further on the filaments, the same web of about 15 g/m² obtained in the above was laminated to obtain a laminate.

The obtained laminate was placed on a mesh-form support having 100 meshes and treated twice by a spray apparatus in which spray nozzles having a pore size of 0.1 mm were arranged transversely in a single line at intervals of 0.6 mm at a spray pressure of 6.9 MPa. Subsequently, the laminate was turned upside down and the rear surface was treated by spray twice at a spray pressure of 9.8 MPa. The laminate was further turned upside down and placed on the mesh-form support having 25 meshes and treated by the spray apparatus twice at a spray pressure of 9.8 MPa. As a result, nonwoven fabric having a weight per unit area of 33 g/m².

### (Examples 62 to 69, 74 to 92 and Comparative Examples 25 to 34)

Nonwoven fabric (weight per unit area: 33 g/m²) was obtained in the same manner as in Example 61 except that the X-ray opaque filament was changed to the X-ray opaque filament or the X-ray opaque covered filament (each of Examples and Comparative Examples) shown in Table 4.

### (Examples 70 and 71)

Nonwoven fabric was obtained in the same manner as in Example 62 except that the weight per unit area of the nonwoven fabric was changed to each of the values shown in Table 4.

### (Examples 72 and 73)

Nonwoven fabric was obtained in the same manner as in Example 62 except that the solvent spun cellulose fiber was changed to viscose rayon fiber B (degree of fineness per single filament: 2.2 dtex, fiber length: 38 mm, Example 72) or cotton C (degree of fineness per single filament: 1.7 dtex, fiber length: 24 mm, Example 73).

Physical property values and evaluations of nonwoven fabric samples of Examples 61 to 92 and Comparative Examples 25 to 34 obtained as mentioned above are shown in Table 4.

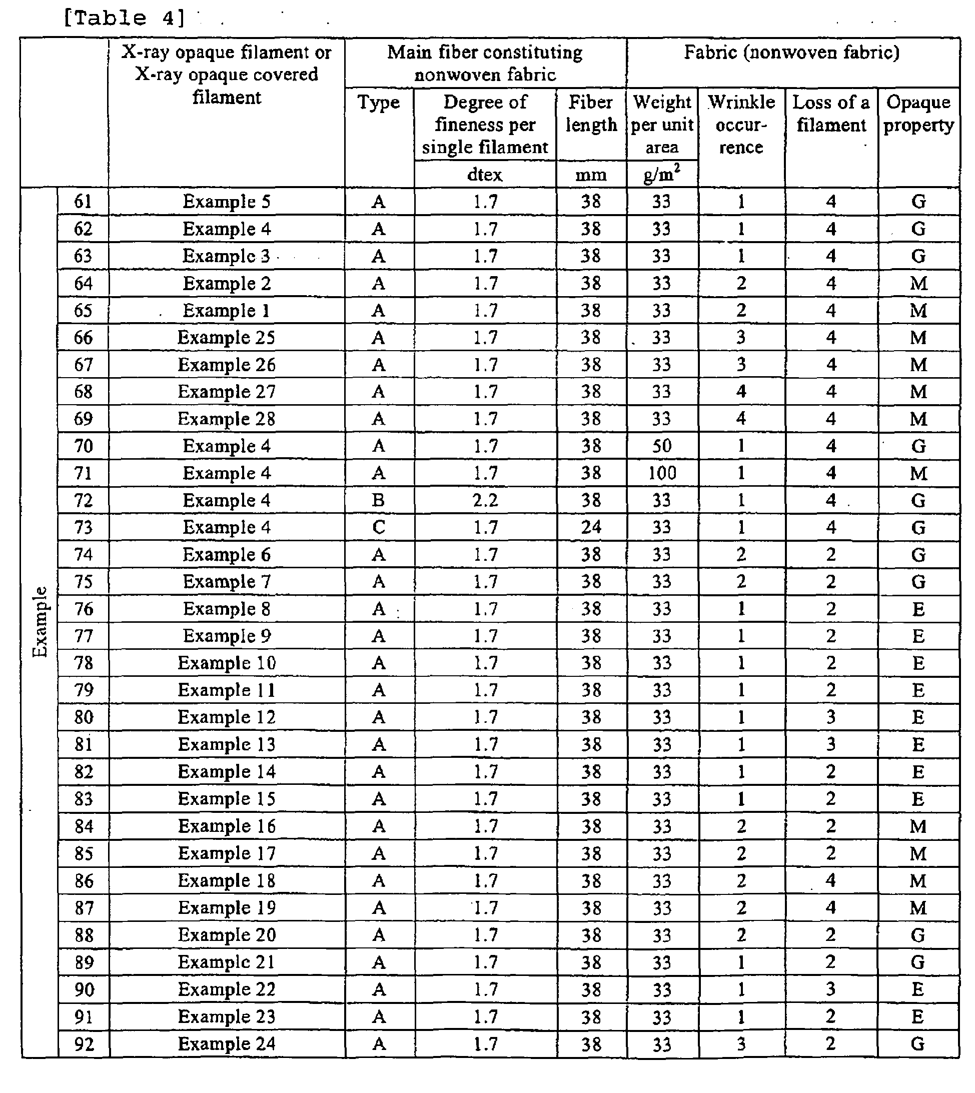

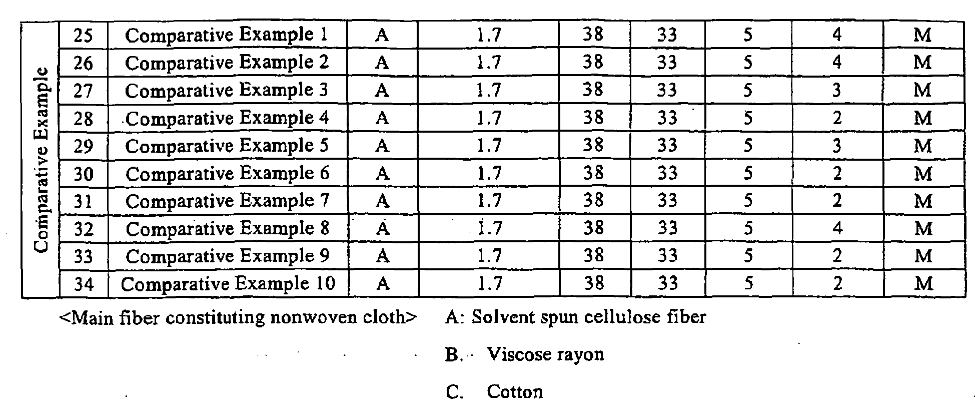

As is apparent from Table 4, in the X-ray opaque filaments or X-ray opaque covered filaments according to Examples 61 to 92, since the dry heat shrinkage of each of the filaments was 3.5% or less, the nonwoven fabric samples obtained had neither wrinkles nor loss of a filament and satisfactory in opaque property. In particular, the X-ray opaque covered filament of each of Examples 74 to 79, 82 to 85, 88, 89, 91 and 92 had little loss of the X-ray opaque filaments since the X-ray opaque filaments were covered. In addition, since the X-ray opaque covered filament were integrally formed such that the sectional shape of a multifilament was substantially circular, the nonwoven cloth samples obtained by using these filaments had more excellent X-ray opaque property.

On the other hand, in each of the X-ray opaque filaments or X-ray opaque covered filaments according to Comparative Examples 25 to 34, since the dry heat shrinkage (at 130°C) of the X-ray opaque filament exceeded 3.5%, the nonwoven fabric samples obtained by using these had many wrinkles and the quality in view of a product was low.

### [Examples of an X-ray opaque covered filament whose covering filament is at least partly formed of a second thermoplastic resin having a lower melting point than a first thermoplastic resin used in an X-ray opaque filament]

### (Covering filament a)

Chips of a nylon copolymer (melting point: 118°C, manufactured by Arkema) consisting of nylon 6, nylon 66 and nylon 12 in a component ratio (by mass) of 42:18:40 were supplied to extruder-type melt spinning machine and spun and extruded from a spinning nozzle having 12 spinning holes of 0.35 mm in diameter at a spinning temperature of 185°C. Drawing was performed by setting first and second roller speeds at 560 m/minute and a final rolling-up speed at 1400 m/minute, so as to obtain a drawing rate of 2.5 fold. The obtained covering filament a had a degree of fineness of 110 dtex/12f as is shown in Table 5.

### (Covering filament b)

Nylon 12 (VESTAMIDL 1900, melting point: 178°C, manufactured by Daicel Degussa Ltd.) having a relative viscosity of 1.90 was employed as a core component, and a nylon copolymer (melting point: 118°C, manufactured by Arkema) consisting of nylon 6, nylon 66 and nylon 12 in a component ratio (by mass) of 42:18:40 was employed as a sheath component. A composite covering filament containing the core component and the sheath component in a mass ratio of 90:10 was spun and extruded from a core/sheath type composite spinning nozzle having 12 spinning holes of 0.35 mm in diameter at a spinning temperature of 250°C. The filament was rolled up by setting a first roller speed at 3000 m/minute, a second roller speed at 3200 m/minute, and a final rolling-up speed at 3500 m/minute. The obtained covering filament had a degree of fineness of 90 dtex/24f, as is shown in Table 5.

### (Covering filaments c and d)

Covering filaments were obtained by melt-spinning in the same manner as in the case of covering filament b except that each of the core to sheath mixing ratio was set at the value shown in Table 5. The results are shown in Table 5.

### (Covering filament e)

Polyethylene terephthalate having a relative viscosity of 0.70 was employed as a core component, and a copolymer of polyethylene terephthalate (melting point: 135°C) having a relative viscosity of 0.68 and isophthalic acid (33.0% by mole) was employed as a sheath component. A conjugate covering filament containing the core component and the sheath component in a mass ratio of 50:50 was spun and extruded from a sheath/core type conjugate spinning nozzle having 24 spinning holes of 0.2 mm in diameter at a spinning temperature of 280°C. The filament was wound up by setting a first godet roller speed at 3000 m/minute (roller temperature: 90°C), a second godet roller speed at 4500 m/minute (roller temperature: 110°C) and a winding up speed at 4500 m/minute. The obtained covering filament had a degree of fineness of 84 dtex/24f, as is shown in Table 5.

### (Covering filament f)

Polyethylene terephthalate (melting point: 260°C) having a relative viscosity of 0.70 was employed as a core component, and polyethylene (melting point: 102°C, melt-flow rate: 20 g/10 minutes) polymerized in the presence of a metallocene based catalyst was employed as a sheath component. A conjugate covering filament containing the core component and the sheath component in a mass ratio 50:50 was spun and extruded from a core/sheath type composite spinning nozzle having 24 spinning holes of 0.2 mm in diameter at a spinning temperature of 280°C and wound up at a winding up speed at 4000 m/minute. The obtained covering filament had a degree of fineness of 84 dtex/24f as is shown in Table 5.

### (Covering filament g)

Nylon 12 (melting point: 178°C) having a relative viscosity of 1.90 used in the case of covering filament b was spun and extruded from 24 spinning holes of 0.35 mm in diameter at a spinning temperature of 250°C. The obtained filament was drawn by setting first and second roller speeds at 560 m/minute and a final winding up speed at 1400 m/minute so as to obtain a drawing ratio of 2.5 fold. The obtained covering filament had a degree of fineness of 90 dtex/24f, as is shown in Table 5.

### (Covering filament h)

Polyethylene terephthalate (melting point: 260°C) having a relative viscosity of 0.70 was spun and extruded from 36 spinning holes of 0.2 mm in diameter at a spinning temperature of 280°C. The obtained filament was wound up by setting a first godet roller speed at 3000 m/minute (roller temperature: 95°C), a second godet roller speed at 4500 m/minute (roller temperature: 130°C) and a winding up speed at 4500 m/minute. The obtained covering filament had a degree of fineness of 84 dtex/36f, as is shown in Table 5.

**[Table 5]**

| | Constituent resin | Melting point | Ratio of Sheath | Degree of fineness | Number of filaments |
|---|---|---|---|---|---|
| | | °C | % by mass | dtex | |
| Covering filament a | Nylon 6/66/12 copolymer | 118 | - | 110 | 12 |
| Covering filament b | Core: Nylon 12 | 178 | 10 | 90 | 24 |
| | Sheath: Nylon 6/66/12 copolymer | 118 | | | |
| Covering filament c | Core: Nylon 12 | 178 | 50 | 90 | 24 |
| | Sheath: Nylon 6/66/12 copolymer | 118 | | | |
| Covering filament d | Core: Nylon 12 | 178 | 80 | 90 | 24 |
| | Sheath: Nylon 6/66/12 copolymer | 118 | | | |
| Covering filament e | Core: Polyethylene terephthalate | 260 | 50 | 84 | 24 |
| | Sheath: IP-copolymerized polyester | 135 | | | |
| Covering filament | Core: polyethylene terephthalate | 260 | 50 | 84 | 24 |
| | f Sheath: polyethylene | 102 | | | |
| Covering filament g | Nylon 12 | 178 | - | 90 | 24 |
| Covering filament h | Polyethylene terephthalate | 260 | - | 84 | 36 |

| | | | | | |
|---|---|---|---|---|---|
| IP: Isophthalic acid | | | | | |

### (Example 93)

The covering filament c was turned around the X-ray opaque filament of Example 5 by use of a covering twister so as to obtain the number of S-shaped twists: 500 T/m to obtain an X-ray opaque covered filament.

### (Examples 94 to 103)

An X-ray opaque covered filament was obtained by covering an X-ray opaque filament with a covering filament in accordance with the conditions shown in Table 6 (as to combinations of X-ray opaque filament of Examples and covering filaments and conditions). Note that, in Examples 96 and 97, X-ray opaque filaments before forming into X-ray opaque covered filaments in Examples 16 and 24, respectively were used. In Example 95, after an X-ray opaque filament was covered with a covering filament, a heating process was performed by use of a slit type heater heated to 130°C for 30 seconds to melt part of the covering filament and solidify it. In this way, the X-ray opaque filament and the covering filament were bonded with heat.

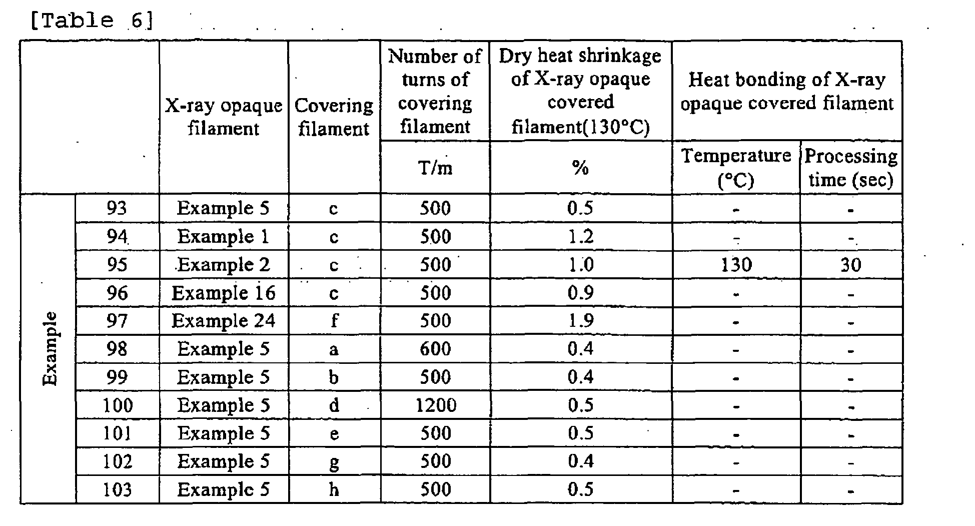

### [Examples of nonwoven fabric containing X-ray opaque covered filament]

### (Example 104)

A fiber web was obtained in the same manner as in Example 61 using solvent spun cellulose fiber A used in Example 61 as a main fiber for constituting nonwoven fabric. Subsequently, on the fiber web, the X-ray opaque covered filaments of Example 93 were arranged linearly at intervals of 100 mm in the machine direction (lengthwise direction). Further on the resultant structure, the same fiber web obtained above was laminated to obtain a laminate.

High pressure water spray treatment was applied to the obtained laminate in the same manner as in Example 61. The fiber sheet obtained by the spray treatment was allowed to pass through a non-contact dry heat processing apparatus. In this manner, thermosetting was performed at 130°C for 30 seconds; at the same time, part of the covering filament was melted to adhere to the main fiber constituting the nonwoven fabric to obtain nonwoven fabric having a weight per unit area of 33 g/m². The physical properties of the nonwoven fabric are shown in Table 7.

### (Examples 105 to 109, 112 to 114)

Nonwoven fabric was obtained in the same manner as in Example 104 except that the type of X-ray opaque covered filament and weight per unit area thereof and the temperature of the thermosetting process were changed to obtain the values shown in Table 7. The physical properties of the obtained nonwoven fabric are shown in Table 7.

### (Examples 110 and 111)

As a main fiber constituting nonwoven fabric, cotton C of Example 73 (Example 100) and viscose rayon fiber B of Example 72 (Example 101) were used. Nonwoven fabric was obtained in the same manner as in example 104 except that types of X-ray opaque covered filaments were changed as shown in Table 7. The physical properties of the obtained nonwoven fabric are shown in Table 7.

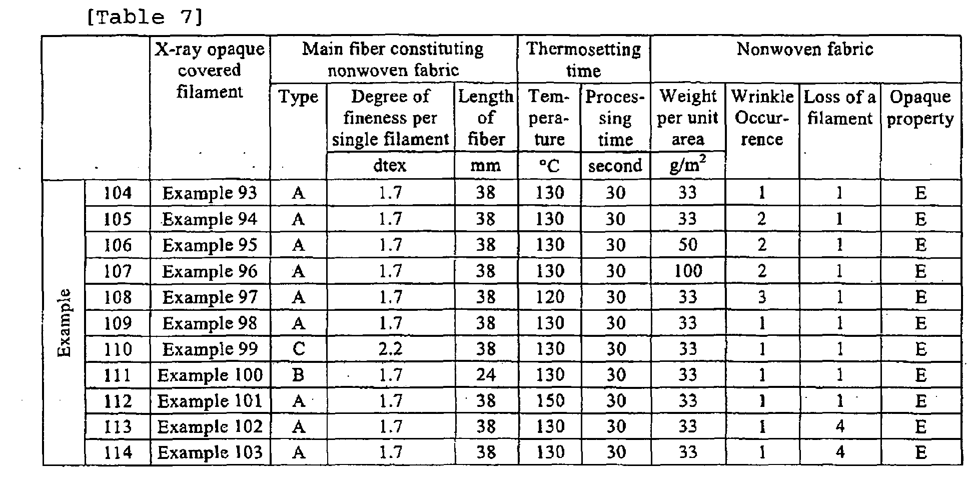

The nonwoven fabric samples obtained in Examples 104 to 114 were not wrinkled and had good quality and excellent in X-ray opaque property. Furthermore, since the covering filament is partly melted to adhere to the X-ray opaque filament and the main fiber constituting nonwoven fabric in each of Examples 104 to 112, the X-ray opaque filament is not pulled out from the nonwoven fabric. Therefore, the evaluation as to loss of an X-ray opaque filament from nonwoven fabric was particularly good.

## Claims

1. An X-ray opaque filament formed of a thermoplastic resin containing an X-ray opaque agent, **characterized in that** the X-ray opaque filament has a dry heat shrinkage of 3.5 to 0 % at 130°C.

2. The X-ray opaque filament according to claim 1, wherein the thermoplastic resin is nylon 12.

3. The X-ray opaque filament according to claim 1, consisting only of the thermoplastic resin containing the X-ray opaque agent.

4. The X-ray opaque filament according to claim 1, wherein the filament is a monofilament having a degree of fineness within 1000 to 20000 dtex.

5. The X-ray opaque filament according to claim 1, wherein the filament is a multifilament having a degree of fineness within 1000 to 20000 dtex and a degree of fineness per single filament within 20 to 400 dtex.

6. The X-ray opaque filament according to anyone of claims 1 to 5, wherein an oil containing an ionic surfactant in a ratio of 0 to 10% by mass is added.

7. An X-ray opaque covered filament wherein an X-ray opaque filament formed of a thermoplastic resin containing an X-ray opaque agent is covered with a covering filament and the X-ray opaque covered filament has a dry heat shrinkage of 3.5 to 0 % at 130°C.

8. The X-ray opaque covered filament according to claim 7, wherein the X-ray opaque filament is one according to any one of claims 1 to 6.

9. The X-ray opaque covered filament according to claim 7, wherein the covering filament has a lower degree of fineness than the X-ray opaque filament.

10. An X-ray opaque covered filament according to claim 7 wherein the X-ray opaque filament according to any one of claims 1 to 6 is used, and the covering filament is at least partly constituted of a second thermoplastic resin having a lower melting point than a first thermoplastic resin forming the X-ray opaque filament.

11. The X-ray opaque covered filament according to claim 10, wherein the melting point of the second thermoplastic resin is 100°C or more and lower by 20°C than the melting point of the first thermoplastic resin.

12. The X-ray opaque covered filament according to claim 10, wherein the covering filament is a conjugate filament formed of a core portion and a sheath portion and the sheath portion of the conjugate filament is formed of the second thermoplastic resin.

13. A fiber structure comprising the X-ray opaque filament according to any one of claims 1 to 6 and/or the X-ray opaque covered filament according to any one of claims 7 to 12.

## Patentansprüche

1. Ein röntgenopakes Filament, das aus einem thermoplastischen Harz hergestellt ist, das ein röntgenopakes Mittel enthält, **dadurch gekennzeichnet, dass** das röntgenopake Filament eine Trockenwärmeschrumpfung von 3,5 bis 0 % bei 130 °C aufweist.

2. Das röntgenopake Filament nach Anspruch 1, wobei das thermoplastische Harz Nylon 12 ist.

3. Das röntgenopake Filament nach Anspruch 1, das nur aus dem thermoplastischen Harz besteht, das das röntgenopake Mittel enthält.

4. Das röntgenopake Filament nach Anspruch 1, wobei das Filament ein Monofilament mit einem Feinheitsgrad innerhalb 1000 bis 20000 dtex ist.

5. Das röntgenopake Filament nach Anspruch 1, wobei das Filament ein Multifilament mit einem Feinheitsgrad innerhalb 1000 bis 20000 dtex und einem Feinheitsgrad pro Einzelfilament innerhalb 20 bis 400 dtex ist.

6. Das röntgenopake Filament nach einem der Ansprüche 1 bis 5, wobei ein Öl zugegeben ist, das ein ionisches Tensid in einem Verhältnis von 0 bis 10 Gew.-% enthält.

7. Ein röntgenopakes ummanteltes Filament, wobei ein röntgenopakes Filament, das aus einem thermoplastischen Harz hergestellt ist, das ein röntgenopakes Mittel enthält, mit einem Mantelfilament ummantelt ist und das röntgenopake ummantelte Filament eine Trockenwärmeschrumpfung von 3,5 bis 0 % bei 130 °C aufweist.

8. Das röntgenopake ummantelte Filament nach Anspruch 7, wobei das röntgenopake Filament ein Filament nach einem der Ansprüche 1 bis 6 ist.

9. Das röntgenopake ummantelte Filament nach Anspruch 7, wobei das Mantelfilament einen geringeren Feinheitsgrad als das röntgenopake Filament aufweist.

10. Ein röntgenopakes ummanteltes Filament nach Anspruch 7, wobei das röntgenopake Filament nach einem der Ansprüche 1 bis 6 verwendet wird und das Mantelfilament mindestens teilweise aus einem zweiten thermoplastischen Harz mit einem geringeren Schmelzpunkt als ein erstes thermoplastisches Harz, das das röntgenopake Filament bildet, zusammengesetzt ist.

11. Das röntgenopake ummantelte Filament nach Anspruch 10, wobei der Schmelzpunkt des zweiten thermoplastischen Harzes 100 °C oder mehr beträgt und um 20 °C geringer als der Schmelzpunkt des ersten thermoplastischen Harzes ist.

12. Das röntgenopake ummantelte Filament nach Anspruch 10, wobei das Mantelfilament ein konjugiertes Filament ist, das aus einem Kernabschnitt und einem Hüllabschnitt gebildet ist, und der Hüllabschnitt des konjugierten Filaments aus dem zweiten thermoplastischen Harz gebildet ist.

13. Eine Faserstruktur, die das röntgenopake Filament nach einem der Ansprüche 1 bis 6 und/oder das röntgenopake ummantelte Filament nach einem der Ansprüche 7 bis 12 umfasst.

## Revendications

1. Filament opaque aux rayons X formé d'une résine thermoplastique contenant un agent opaque aux rayons X, **caractérisé en ce que** le filament opaque aux rayons X présente un retrait à la chaleur sèche de 3,5 à 0% à 130°C.

2. Filament opaque aux rayons X selon la revendication 1, dans lequel la résine thermoplastique est du nylon 12.

3. Filament opaque aux rayons X selon la revendication 1, ne consistant qu'en la résine thermoplastique contenant l'agent opaque aux rayons X.

4. Filament opaque aux rayons X selon la revendication 1, dans lequel le filament est un monofilament ayant un degré de finesse d'entre 1 000 et 20 000 dtex.

5. Filament opaque aux rayons X selon la revendication 1, dans lequel le filament est un multifilament ayant un degré de finesse d'entre 1 000 et 20 000 dtex et un degré de finesse par filament unique d'entre 20 à 400 dtex.

6. Filament opaque aux rayons X selon l'une quelconque des revendications 1 à 5, dans lequel une huile contenant un agent tensioactif ionique dans un rapport de 0 à 10% en masse est ajoutée.

7. Filament recouvert opaque aux rayons X dans lequel un filament opaque aux rayons X formé d'une résine thermoplastique contenant un agent opaque aux rayons X est recouvert avec un filament de recouvrement et le filament recouvert opaque aux rayons X présente un retrait à la chaleur sèche de 3,5 à 0% à 130°C.

8. Filament recouvert opaque aux rayons X selon la revendication 7, dans lequel le filament opaque aux rayons X est un filament selon l'une quelconque des revendications 1 à 6.

9. Filament recouvert opaque aux rayons X selon la revendication 7, dans lequel le filament de recouvrement présente un degré de finesse plus petit que le filament opaque aux rayons X.

10. Filament recouvert opaque aux rayons X selon la revendication 7, dans lequel le filament opaque aux rayons X selon l'une quelconque des revendications 1 à 6 est utilisé, et le filament de recouvrement est au moins partiellement constitué d'une deuxième résine thermoplastique ayant un point de fusion inférieur à celui d'une première résine thermoplastique formant le filament opaque aux rayons X.

11. Filament recouvert opaque aux rayons X selon la revendication 10, dans lequel le point de fusion de la deuxième résine thermoplastique est 100°C ou plus et inférieur de 20°C au point de fusion de la première résine thermoplastique.

12. Filament recouvert opaque aux rayons X selon la revendication 10, dans lequel le filament de recouvrement est un filament conjugué formé d'une partie de coeur et d'une partie de gaine et la partie de gaine du filament conjugué est formée de la deuxième résine thermoplastique.

13. Structure fibreuse comprenant le filament opaque aux rayons X selon l'une quelconque des revendications 1 à 6 et/ou le filament recouvert opaque aux rayons X selon l'une quelconque des revendications 7 à 12.
